(19) Europäisches Patentamt
European Patent Office
Office européen des brevets

(11) EP 2 144 880 B1

(12) # EUROPÄISCHE PATENTSCHRIFT

(45) Veröffentlichungstag und Bekanntmachung des Hinweises auf die Patenterteilung:
**30.05.2012 Patentblatt 2012/22**

(21) Anmeldenummer: **08736070.7**

(22) Anmeldetag: **10.04.2008**

(51) Int Cl.:
***C07D 215/24*** *(2006.01)* ***C11D 3/39*** *(2006.01)*
***C11D 3/16*** *(2006.01)*

(86) Internationale Anmeldenummer:
**PCT/EP2008/054349**

(87) Internationale Veröffentlichungsnummer:
**WO 2008/135337 (13.11.2008 Gazette 2008/46)**

(54) **BIS(HYDROXYCHINOLIN)-METALLKOMPLEXE ALS BLEICHKATALYSATOREN**

BIS(HYDROXYQUINOLINE) METAL COMPLEXES AS BLEACH CATALYSTS

COMPLEXES MÉTALLIQUES DE BIS(HYDROXYQUINOLINE) EN TANT QUE CATALYSEURS DE BLANCHIMENT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.04.2007 DE 102007017654**

(43) Veröffentlichungstag der Anmeldung:
**20.01.2010 Patentblatt 2010/03**

(73) Patentinhaber: **Henkel AG & Co. KGaA**
**40589 Düsseldorf (DE)**

(72) Erfinder:
- **HÄTZELT, Andre**
  **40591 Düsseldorf (DE)**
- **NORDSKOG, Anette**
  **40477 Düsseldorf (DE)**
- **LEOPOLD, Stefan**
  **40589 Düsseldorf (DE)**
- **SCHMIEDEL, Peter**
  **40599 Düsseldorf (DE)**
- **RYBINSKI VON, Wolfgang**
  **40593 Düsseldorf (DE)**
- **SUNDERMEYER, Jörg**
  **35041 Marburg (DE)**
- **DÖRING, Jan**
  **35039 Marburg (DE)**

(56) Entgegenhaltungen:
**EP-A- 0 964 459 DE-A1- 2 054 019**

- **FAGAN ET AL.: "Using Intelligent/Random Library Screening To Desigh Focused Libraries for the Optimization of Homogeneous Catalysts: Ullmann Ether Formation" JOURNAL OF THE AMERICAN CHEMICAL SOCIETY, Bd. 122, 5. Oktober 2000 (2000-10-05), Seiten 5043-5051, XP002484006 Washington,DC, US in der Anmeldung erwähnt**
- **STOCKWELL B R ET AL: "Chemical Genetic and Genomic Approaches Reveal a Role for Copper in specific Gene Activation" JOURNAL OF THE AMERICAN CHEMICAL SOCIETY, AMERICAN CHEMICAL SOCIETY, WASHINGTON, DC, US, Bd. 121, 30. Oktober 1999 (1999-10-30), Seiten 10662-10663, XP002381594 ISSN: 0002-7863 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Bis(hydroxychinolin)-Metallkomplexe sowie deren Verwendung als Bleichkatalysatoren.

Zur effektiven Bleiche mit Wasserstoffperoxid muss dieses in eine bleichaktivere Spezies umgewandelt werden. Eine Möglichkeit zur Erzeugung aktivierter Peroxy-Verbindungen besteht in der Verwendung von Persäurevorläufern, sogenannter Bleichaktivatoren wie z.B. TAED, die durch Perhydrolyse in die aktive Spezies umgewandelt werden.

Eine weitere Möglichkeit zur Erzeugung aktivierter Spezies besteht in der enzymatisch katalysierten Perhydrolyse von Carbonsäureestern oder Nitrilverbindungen unter Verwendung von Perhydrolasen.

Schließlich ist es auch bekannt, Bleichkatalysatoren zur Erzeugung aktivierter Spezies zu verwenden, wobei unter einem Bleichkatalysator ein Stoff zu verstehen ist, der die Bleichleistung von Wasserstoffperoxid an einem bleichbaren Stoff verbessern kann, ohne selbst stöchiometrisch an der Reaktion beteiligt zu sein.

Die Verwendung von Bleichkatalysatoren hat gegenüber den anderen Verfahren der Bleichaktivierung den Vorteil, dass substöchiometrische Mengen der Verbindung ausreichen, wodurch in der Formulierung des bleichehaltigen Produkts eine Raum- und Gewichtsersparnis erreicht werden kann. Weiterhin ist mit der Reduzierung des Gewichts, insbesondere bei Wasch-und Reinigungsanwendungen, auch der Vorteil verknüpft, dass es zu einem geringeren Stoffeintragung in die Umwelt kommt, was aus ökologischen Gründen besonders vorteilhaft ist. Daneben können hierdurch auch Transport- und Verpackungskosten eingespart werden.

Weiterhin ist zu berücksichtigen, dass es bei Verwendung von Bleichaktivatoren wie Nitrilen oder TAED in Gegenwart von Wasser zu einer vorzeitigen Hydrolyse kommen kann, während dieses Problem bei Verwendung von Bleichkatalysatoren weitestgehend vermieden werden kann. Darüber hinaus verursacht die bei der nichtkatalytischen Bleichaktivierung ausgehend von den Persäuren erfolgende Entstehung von Säuren eine Verschiebung des pH-Wertes, die sich ungünstig auf die Bleichleistung auswirken kann. Des Weiteren ist die Bleichleistung der meisten Bleichaktivatoren bei niedrigen Temperaturen oft unzureichend.

Aus den oben genannten Gründen ist die Verwendung von Bleichkatalysatoren von besonderem Interesse gegenüber den anderen Techniken der Bleichaktivierung, so dass grundsätzlich Bedarf an neuen Bleichkatalysatoren besteht.

Als Bleichkatalysatoren sind insbesondere Metall-Komplexe von organischen Liganden wie Salenen, Saldiminen, Tris[salicylidenaminoethyl]aminen, monocyclischen Polyazaalkanen, querverbrückten polycyclischen Polyazaalkanen, Terpyridinen und Tetraamido-Liganden beschrieben. Ein Nachteil der beschriebenen Metallkomplexe besteht jedoch darin, dass sie entweder, insbesondere bei niedriger Temperatur, keine ausreichende Bleichleistung besitzen oder es aber bei ausreichender Bleichleistung zu einer unerwünschten Schädigung von Farben und gegebenenfalls auch der Textilfasern kommt.

Überraschenderweise wurde nun gefunden, dass Bis(hydroxychinolin)-Metallkomplexe hervorragend als Oxidationskatalysator geeignet sind und sich gleichzeitig schonender gegenüber Wäsche verhalten als die heute gebräuchlichen Bleichkatalysatoren.

N-Alkyl-2,2'-imino-bis-(8-hydroxychinolin)-Metallkomplexe sind im Stand der Technik bereits beschrieben. So beschreibt DE19825737 Zink-, Magnesium-, Aluminium-, Gallium-, Indium- und Lutetium-Komplexe derartiger Liganden sowie die Verwendung dieser Komplexe in einer elektrolumineszierenden Anordnung. Agustin et al. (J. Organomet. Chem. 592 (1999) 1-10) beschreiben Germanium- und Zinn-Komplexe von N-Methyl-2,2'-imino-bis(8-hydroxychinolin) und Fagan et al. (J. Am. Chem. Soc. 122 (2000) 5043-5051) beschreiben den Kupfer-Komplex dieses Liganden sowie dessen Untersuchung auf die Eigenschaft hin, 2-Brom-4,6-dimethylaniline zu phenoxylieren. Stockwell et al. (J. Am. Chem. Soc. 121 (1999) 10662-10663) untersuchen den Einfluss von N-Methyl-2,2'-imino-bis(8-hydroxychinolin) auf die Expression unterschiedlicher Gene in Saccharomyces cerevisiae und untersuchen außerdem die Komplexbildungskonstante dieses Liganden in Bezug auf unterschiedliche Metallionen.

Wang et al. (Inorganica Chimica Acta 321 (2001) 215-220) beschreiben Ruthenium-Komplexe des Bis(8-hydroxychinolin-2-yl)ethers. In DE2650764 wird die Verwendung von 2,2'-Methylen-bis(8-hydroxychinolin) als polyfunktioneller Kuppler zur Entwicklung von Farbbildern beschrieben.

Die Verwendung der Liganden und Metall-Ligand-Komplexe als Bleichkatalysatoren oder als Zusatzstoff für Wasch- und Reinigungsmittel wird in den genannten Dokumenten nicht beschrieben.

Ein erster Gegenstand der vorliegenden Erfindung sind daher Wasch- und Reinigungsmittel enthaltend Liganden und/oder Metall-Ligand-Komplexe von Liganden der allgemeinen Formel (I)

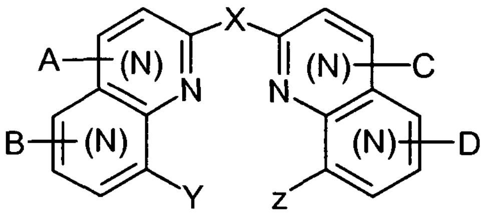

(I),

wobei (N) bedeutet, dass optional ein oder zwei CH-Gruppen des entsprechenden Arylrests durch N ersetzt sein können.

Die Grundstruktur kann hierbei insbesondere ausgewählt sein aus

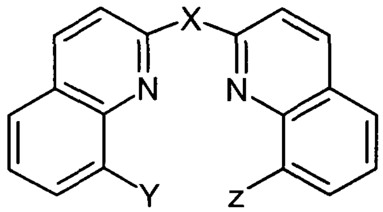
,
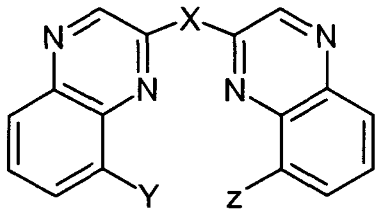
,
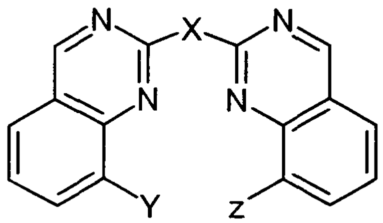
,
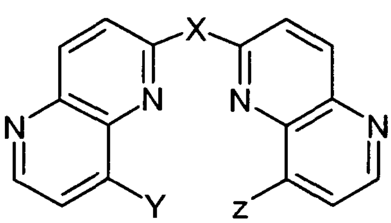
,
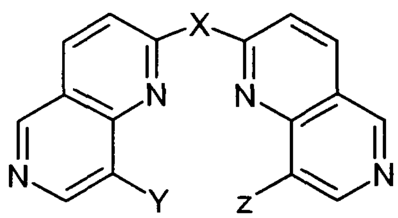
,
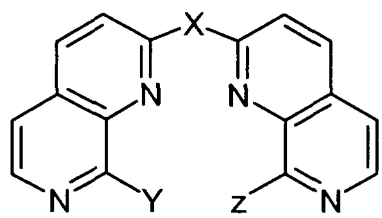
,
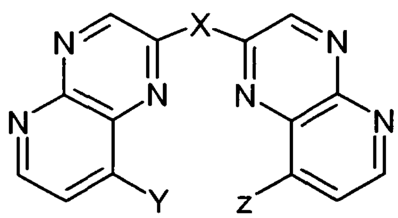
,
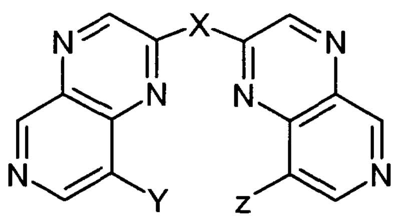
,
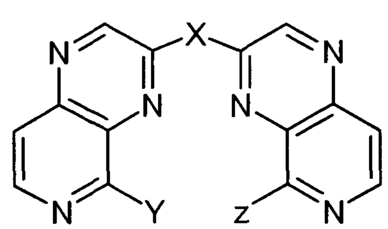
,
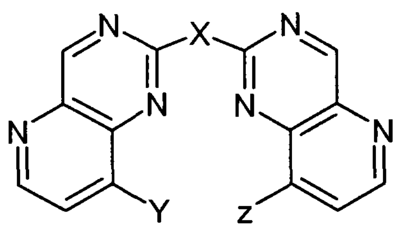
,

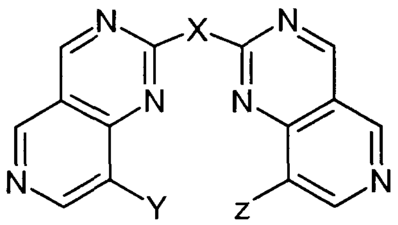,
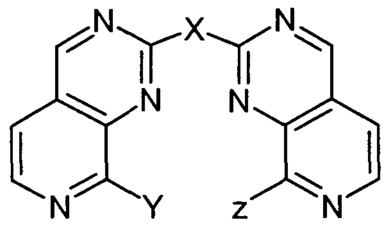,

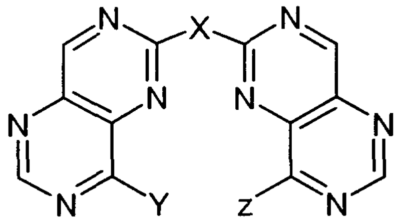,
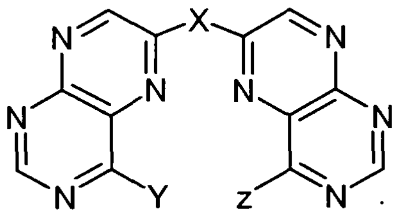.

Bevorzugt sind Liganden der allgemeinen Formel (II)

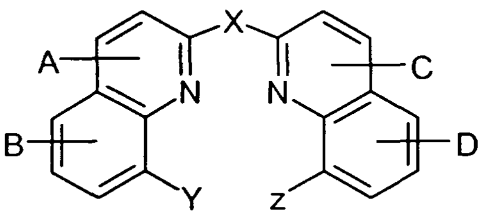
(II)

Besonders bevorzugt sind Bishydroxychinoline der allgemeinen Formel (III)

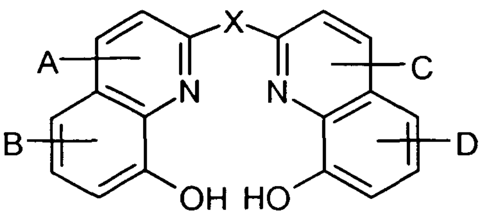
(III)

In den Formeln stehen
X für $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ oder $SiR^3R^4$,
Y und Z unabhängig voneinander für OH, SH, $NH_2$, $NHR^5$ oder $NR^5R^6$,
A, B, C, D, $R^1$, $R^2$, $R^3$ und $R^4$ unabhängig voneinander für Wasserstoff, Alkyl, insbesondere $C_{1-22}$-Alkyl, vorzugsweise $C_{1-18}$-Alkyl, Trifluormethyl, Cycloalkyl, insbesondere $C_{3-8}$-Cycloalkyl, Cycloalkylalkyl, insbesondere $C_{3-8}$-Cycloalkyl-$C_{1-12}$-alkyl, Alkenyl, insbesondere $C_{2-18}$-Alkenyl, Alkinyl, insbesondere $C_{2-18}$-Alkinyl, Heteroalkyl, Heterocycloalkyl, Alkoxy, insbesondere $C_{1-18}$-Alkoxy, Alkylsulfanyl, insbesondere $C_{1-18}$-Alkylsulfanyl, Alkylsulfinyl, insbesondere $C_{1-18}$-Alkylsulfinyl, Alkylsulfonyl, insbesondere $C_{1-18}$-Alkylsulfonyl , Alkanoyl, insbesondere $C_{1-18}$-Alkanoyl, Alkanoyloxy, insbesondere $C_{1-18}$-Alkanoyloxy, Alkoxycarbonyl, insbesondere $C_{1-18}$-Alkoxycarbonyl, Alkylaminocarbonyl, insbesondere $C_{1-18}$-Alkylaminocarbonyl, Alkylsulfanylcarbonyl, insbesondere $C_{1-18}$-Alkylsulfanylcarbonyl, Hydroxy, Amino, Aryl, insbesondere $C_{6-10}$-Aryl, Arylalkyl, insbesondere $C_{6-10}$-Aryl-$C_{1-12}$-alkyl, Aryloxy, insbesondere $C_{6-10}$-Aryloxy, Arylsulfanyl, insbesondere $C_{6-10}$-Arylsulfanyl, Arylsulfinyl, insbesondere $C_{6-10}$-Arylsulfinyl, Arylsulfonyl, insbesondere $C_{6-10}$-Arylsulfonyl, Arylcarbonyl, insbesondere $C_{6-10}$-Arylcarbonyl, Arylcarbonyloxy, insbesondere $C_{6-10}$-Arylcarbonyloxy, Aryloxycarbonyl, insbesondere $C_{6-10}$-Aryloxycarbonyl, Arylaminocarbonyl, insbesondere $C_{6-10}$-Arylaminocarbonyl, Arylsulfanylcarbonyl, insbesondere $C_{6-10}$-Arylsulfanylcarbonyl, Heteroaryl, Heteroarylalkyl, insbesondere Heteroaryl-$C_{1-12}$-alkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl, Heteroarylsulfanylcarbonyl, Alkoxysulfonyl, insbesondere $C_{1-18}$-Alkoxysulfonyl, Alkoxycarbinol, insbesondere $C_{1-12}$-Alkoxycarbinol, Ammonium, Hydroxycarbonyl, Alk-

oxycarbonyl, insbesondere $C_{1-18}$-Alkoxycarbonyl, Aryloxycarbonyl, insbesondere $C_{6-10}$-Aryloxycarbonyl, Amidocarbonyl, Halogen, insbesondere Chlor, Brom, Iod oder Fluor, Nitro, Sulfato, Sulfo, Amidosulfo, Phosphato, Phosphono, Amidophosphono, Formyl, Thioformyl, $-(CH_2-CH_2-O-)_nH$ und $-(CH_2-CH_2-CH_2-O)_nH$ mit n = 1 bis 20, vorzugsweise 3 bis 20,
$R^5$ und $R^6$ unabhängig voneinander für Wasserstoff oder Alkyl, insbesondere $C_{1-18}$-Alkyl, wobei alle Reste des sich so ergebenden Moleküls, insbesondere die aliphatischen und aromatischen Reste jeweils unabhängig voneinander gegebenenfalls auch ein- oder mehrfach, insbesondere ein-, zwei- oder dreifach, vorzugsweise einfach, substituiert sein können, insbesondere durch Substituenten ausgewählt aus den zuvor genannten Resten.

In einer bevorzugten Ausführungsform haben A, B, C, D, $R^3$, $R^4$, $R^5$ und $R^6$ die zuvor genannten Bedeutungen und stehen $R^1$ und $R^2$ unabhängig voneinander für Wasserstoff, Alkyl, insbesondere $C_{1-22}$-Alkyl, vorzugsweise $C_{1-18}$-Alkyl, Cycloalkyl, insbesondere $C_{3-8}$Cycloalkyl, Cycloalkylalkyl, insbesondere $C_{3-8}$-Cycloalkyl-$C_{1-12}$-alkyl, Alkenyl, insbesondere $C_{2-18}$-Alkenyl, Alkinyl, insbesondere $C_{2-18}$-Alkinyl, Heteroalkyl, Heterocycloalkyl, Alkanoyl, insbesondere $C_{1-18}$-Alkanoyl, Alkoxycarbonyl, insbesondere $C_{1-18}$-Alkoxycarbonyl, Alkylaminocarbonyl, insbesondere $C_{1-18}$-Alkylaminocarbonyl, Alkylsulfanylcarbonyl, insbesondere $C_{1-18}$-Alkylsulfanylcarbonyl, Aryl, insbesondere $C_{6-10}$-Aryl, Arylalkyl, insbesondere $C_{6-10}$-Aryl-$C_{1-12}$-alkyl, Arylcarbonyl, insbesondere $C_{6-10}$-Arylcarbonyl, Aryloxycarbonyl, insbesondere $C_{6-10}$-Aryloxycarbonyl, Arylaminocarbonyl, insbesondere $C_{6-10}$-Arylaminocarbonyl, Arylsulfanylcarbonyl, insbesondere $C_{6-10}$-Arylsulfanylcarbonyl, Heteroaryl, Heteroarylalkyl, insbesondere Heteroaryl-$C_{1-12}$-alkyl, Heteroarylcarbonyl, Heteroaryloxycarbonyl, Heteroarylaminocarbonyl, Heteroarylsulfanylcarbonyl, Trifluormethyl, Formyl, $-(CH_2-CH_2-O-)_nH$ oder $-(CH_2-CH_2-CH_2-O)_nH$ mit n = 1 bis 20,
wobei alle Reste des sich so ergebenden Moleküls, insbesondere die aliphatischen und aromatischen Reste, jeweils unabhängig voneinander gegebenenfalls auch ein- oder mehrfach, insbesondere ein-, zwei- oder dreifach, vorzugsweise einfach, substituiert sein können, insbesondere durch Substituenten ausgewählt aus den zuvor genannten Resten sowie ausgewählt aus Ammonium, Hydroxycarbonyl, Alkoxycarbonyl, insbesondere $C_{1-18}$-Alkoxycarbonyl, Aryloxycarbonyl, insbesondere $C_{6-10}$-Aryloxycarbonyl, Amidocarbonyl, Halogen, insbesondere Chlor, Brom, Iod oder Fluor, Nitro, Sulfato, Sulfo, Amidosulfo, Phosphato, Phosphono, Amidophosphono, Hydroxy, Alkoxy, insbesondere $C_{1-18}$-Alkoxy, Amino und Alkanoyloxy, insbesondere $C_{1-18}$-Alkanoyloxy.

In einer weiteren bevorzugten Ausführungsform steht X für $NR^1$, $NR^1R^{2(+)}$, $PR^1$ oder $PR^1R^{2(+)}$ und haben A, B, C, D, Y, Z, $R^1$, $R^2$, $R^3$, $R^4$, $R^5$ und $R^6$ die zuvor genannte Bedeutung.

In einer weiteren bevorzugten Ausführungsform stehen Y und Z für OH und haben A, B, C, D, X, $R^1$, $R^2$, $R^3$, $R^4$, $R^5$ und $R^6$ die zuvor genannten Bedeutung.

In einer besonders bevorzugten Ausführungsform stehen
X für $NR^1$, $NR^1R^{2(+)}$, $PR^1$, $PR^1R^{2(+)}$ oder S,
Y und Z für OH,
$R^1$ und $R^2$ unabhängig voneinander für Wasserstoff, $C_{1-8}$-Alkyl, $-(CH_2-CH_2-O-)_nH$ oder $-(CH_2-CH_2-CH_2-O)_nH$ mit n = 1 bis 20,
A, B, C und D unabhängig voneinander für Wasserstoff, Alkyl, insbesondere $C_{1-18}$-Alkyl, Ammonium, Hydroxycarbonyl, Alkoxycarbonyl, insbesondere $C_{1-18}$-Alkoxycarbonyl, Aryloxycarbonyl, insbesondere $C_{6-10}$-Aryloxycarbonyl, Amidocarbonyl, Halogen, insbesondere Chlor, Brom, Iod oder Fluor, Nitro, Sulfato, Sulfo, Amidosulfo, Phosphato, Phosphono, Amidophosphono, Hydroxy, Alkoxy, insbesondere $C_{1-18}$-Alkoxy, oder Amino, wobei alle Reste des sich so ergebenden Moleküls jeweils unabhängig voneinander gegebenenfalls auch ein- oder mehrfach, insbesondere ein-, zwei- oder dreifach, vorzugsweise einfach, substituiert sein können, insbesondere durch Substituenten ausgewählt aus den zuvor genannten Resten.

In einer weiteren bevorzugten Ausführungsform umfasst mindestens einer der Reste A, B, C, D und X mindestens eine Gruppe ausgewählt aus Ammonium, Nitro, Sulfato, Sulfo, Amidosulfo, Hydroxycarbonyl, Alkoxycarbonyl, Aryloxycarbonyl, Amidocarbonyl, Phosphato, Phosphono, Amidophosphono, Hydroxy, Alkoxy, Amino, Polyoxyethylen und Halogen als Substituenten, wobei der Rest hierbei insbesondere ausgewählt sein kann aus Sulfo, Sulfoalkyl, insbesondere Sulfo-$C_{1-18}$-Alkyl, Hydroxycarbonyl, Hydroxycarbonylalkyl, insbesondere Hydroxycarbonyl-$C_{1-18}$-alkyl, Phosphono, Phosphonoalkyl, insbesondere Phosphono-$C_{1-18}$-alkyl Hydroxy, Hydroxyalkyl, insbesondere Hydroxy-$C_{1-18}$-alkyl, Amino, Aminoalkyl, insbesondere Amino-$C_{1-18}$-alkyl, Halogen, Haloalkyl, insbesondere Halo-$C_{1-18}$-alkyl, $-(CH_2-CH_2-O-)_nH$ und $C_{1-18}$-alkyl-$(CH_2-CH_2-O-)_nH$ mit jeweils n = 1 bis 20, vorzugsweise 3 bis 20.

$C_{1-18}$-Alkyl steht erfindungsgemäß jeweils unabhängig voneinander für alle gesättigten linearen und verzweigten Alkyl-Reste mit bis zu 18 C-Atomen, wobei $C_{1-8}$-Alkyl-Reste bevorzugt sind. $C_{1-6}$-Alkyl steht erfindungsgemäß für alle gesättigten linearen und verzweigten Alkyl-Reste mit bis zu 6 C-Atomen, insbesondere für Methyl, Ethyl, n-Propyl, i-Propyl, n-Butyl, i-Butyl, t-Butyl sowie alle Isomere des Pentyl und des Hexyl.

$C_{3-8}$-Cycloalkyl steht erfindungsgemäß jeweils unabhängig voneinander für alle cyclischen Alkyl-Reste mit 3 bis 8 C-Atomen, vorzugsweise mit 5 bis 6 C-Atomen, wobei die Reste gesättigt oder ungesättigt sein können, insbesondere für Cyclopentyl, Cyclohexyl oder Cyclopentadienyl.

$C_{2-18}$-Alkenyl steht erfindungsgemäß jeweils unabhängig voneinander für alle linearen und verzweigten Alkyl-Reste mit bis zu 18 C-Atomen, die mindestens eine Doppelbindung enthalten, wobei $C_{2-6}$-Alkenyl-Reste bevorzugt sind. $C_{2-6}$-Alkenyl steht erfindungsgemäß für alle linearen und verzweigten Alkyl-Reste mit bis zu 6 C-Atomen, die mindestens eine Doppelbindung enthalten, insbesondere für Ethenyl, Propenyl, i-Propenyl sowie alle Isomere des Butenyl, Pentenyl und Hexenyl.

$C_{2-18}$-Alkinyl steht erfindungsgemäß jeweils unabhängig voneinander für alle linearen und unverzweigten Alkyl-Reste mit bis zu 18 C-Atomen, die mindestens eine Dreifachbindung enthalten, wobei $C_{2-6}$-Alkinyl-Reste bevorzugt sind. $C_{2-6}$-Alkinyl steht erfindungsgemäß für alle linearen und unverzweigten Alkyl-Reste mit bis zu 6 C-Atomen, die mindestens eine Dreifachbindung enthalten, insbesondere für Ethinyl, Propinyl, i-Propinyl sowie alle Isomere des Butinyl, Pentinyl und Hexinyl.

Heteroalkyl steht erfindungsgemäß jeweils unabhängig voneinander für alle gesättigten und ein-oder mehrfach ungesättigten, linearen oder verzweigten Alkyl-Reste, die mindestens ein, bevorzugt genau ein Heteroatom, insbesondere ausgewählt aus O, S und N, enthalten, wobei die Summe aus C- und Hetero-Atomen bevorzugt bis zu 18, besonders bevorzugt bis zu 6, beträgt.

Heterocycloalkyl steht erfindungsgemäß jeweils unabhängig voneinander für alle cyclischen Alkyl-Reste, die mindestens ein, bevorzugt genau ein, Heteroatom, insbesondere ausgewählt aus O, S oder N, enthalten, wobei der Ring vorzugsweise drei- bis achtgliederig, besonders bevorzugt fünfbis sechsgliedrig ist. Beispiele hierfür sind Tetrahydrofuranyl, Tetrahydrothiophenyl, Pyrrolidinyl, 2-Thiazolinyl, Tetrahydrothiazolyl, Tetrahydrooxazolyl, Piperidinyl, Piperazinyl, Morpholinyl und Thiomorpholinyl.

$C_{1-18}$-Alkoxy steht erfindungsgemäß jeweils unabhängig voneinander für alle gesättigten und ungesättigten, linearen und verzweigten Alkyl-Reste mit bis zu 18 C-Atomen, die über ein Sauerstoff-Atom gebunden sind, wobei $C_{1-6}$-Alkoxy-Reste bevorzugt sind. $C_{1-6}$-Alkoxy steht erfindungsgemäß jeweils unabhängig voneinander für alle gesättigten und ungesättigten, linearen und verzweigten Alkyl-Reste mit bis zu 6 C-Atomen, die über ein Sauerstoff-Atom gebunden sind, insbesondere für Methoxy und Ethoxy.

$C_{1-18}$-Alkylsulfanyl steht erfindungsgemäß jeweils unabhängig voneinander für alle gesättigten und ungesättigten, linearen und verzweigten Alkyl-Reste mit bis zu 18 C-Atomen, die über ein Schwefel-Atom gebunden sind, wobei $C_{1-6}$-Alkylsulfanyl-Reste bevorzugt sind. $C_{1-6}$-Alkylsulfanyl steht erfindungsgemäß für alle gesättigten und ungesättigten, linearen und verzweigten Alkyl-Reste mit bis zu 6 C-Atomen, die über ein Schwefel-Atom gebunden sind, insbesondere für Methysulfanyl und Ethylsulfanyl.

$C_{1-18}$-Alkylsulfinyl steht erfindungsgemäß jeweils unabhängig voneinander für alle gesättigten und ungesättigten, linearen und verzweigten Alkyl-Reste mit bis zu 18 C-Atomen, die über eine SO-Gruppe gebunden sind, wobei $C_{1-6}$-Alkylsulfonyl-Reste bevorzugt sind. $C_{1-6}$-Alkylsulfinyl steht erfindungsgemäß für alle gesättigten und ungesättigten, linearen und verzweigten Alkyl-Reste mit bis zu 6 C-Atomen, die über eine SO-Gruppe gebunden sind, insbesondere für Methylsulfinyl und Ethylsulfinyl.

$C_{1-18}$-Alkylsulfonyl steht erfindungsgemäß jeweils unabhängig voneinander für alle gesättigten und ungesättigten, linearen und verzweigten Alkyl-Reste mit bis zu 18 C-Atomen, die über eine $SO_2$-Gruppe gebunden sind, wobei $C_{1-6}$-Alkylsulfoxidyl-Reste bevorzugt sind. $C_{1-6}$-Alkylsulfonyl steht erfindungsgemäß für alle gesättigten und ungesättigten, linearen und verzweigten Alkyl-Reste mit bis zu 6 C-Atomen, die über eine $SO_2$-Gruppe gebunden sind, insbesondere für Methylsulfonyl und Ethylsulfonyl.

$C_{1-18}$-Alkanoyl steht erfindungsgemäß jeweils unabhängig voneinander für alle gesättigten und ungesättigten, linearen und verzweigten Alkyl-Reste mit bis zu 18 C-Atomen, die über eine Carbonyl-Gruppe gebunden sind, wobei $C_{1-6}$-Alkanoyl-Reste bevorzugt sind. $C_{1-6}$-Alkanoyl steht erfindungsgemäß für alle gesättigten und ungesättigten, linearen und verzweigten Alkyl-Reste mit bis zu 6 C-Atomen, die über eine Carbonyl-Gruppe gebunden sind, insbesondere für Methycarbonyl und Ethylcarbonyl.

$C_{1-18}$-Alkanoyloxy steht erfindungsgemäß jeweils unabhängig voneinander für alle gesättigten und ungesättigten, linearen und verzweigten Alkyl-Reste mit bis zu 18 C-Atomen, die über eine Carbonyloxy-Gruppe gebunden sind, wobei $C_{1-6}$-Alkanoyloxy-Reste bevorzugt sind. $C_{1-6}$-Alkanoyloxy steht erfindungsgemäß für alle gesättigten und ungesättigten, linearen und verzweigten Alkyl-Reste mit bis zu 6 C-Atomen, die über eine Carbonyloxy-Gruppe gebunden sind, insbesondere für Methanoyloxy, Ethanoyloxy, n-Propanoyloxy und i-Propanoyloxy.

$C_{1-18}$-Alkoxycarbonyl steht erfindungsgemäß jeweils unabhängig voneinander für alle gesättigten und ungesättigten, linearen und verzweigten Alkyl-Reste mit bis zu 18 C-Atomen, die über eine Oxycarbonyl-Gruppe gebunden sind, wobei $C_{1-6}$-Alkoxycarbonyl-Reste bevorzugt sind. $C_{1-6}$-Alkoxycarbonyl steht erfindungsgemäß für alle gesättigten und ungesättigten, linearen und verzweigten Alkyl-Reste mit bis zu 6 C-Atomen, die über eine Oxycarbonyl-Gruppe gebunden sind, insbesondere für Methoxycarbonyl und Ethoxycarbonyl.

$C_{1-18}$-Alkylaminocarbonyl steht erfindungsgemäß jeweils unabhängig voneinander für eine Aminocarbonyl-Gruppe, die ein- oder zweifach durch einen gesättigten oder ungesättigten, linearen oder verzweigten Alkyl-Rest mit bis zu 18 C-Atomen substituiert ist, wobei ein- oder zweifach durch $C_{1-6}$-Alkyl-Gruppen substituierte Aminocarbonyl-Reste, insbesondere Monomethylaminocarbonyl, Diemethylaminocarbonyl, Monoethylaminocarbonyl und Diethylaminocarbonyl, bevorzugt sind.

$C_{1-18}$-Alkylsulfanylcarbonyl steht erfindungsgemäß jeweils unabhängig voneinander für alle gesättigten und ungesättigten, linearen und verzweigten Alkyl-Reste mit bis zu 18 C-Atomen, die über eine Thiocarbonyl-Gruppe gebunden sind, wobei $C_{1-6}$-Alkylsulfanylcarbonyl-Reste bevorzugt sind. $C_{1-6}$-Alkylsulfanylcarbonyl steht erfindungsgemäß für alle gesättigten und ungesättigten, linearen und verzweigten Alkyl-Reste mit bis zu 6 C-Atomen, die über eine Thiocarbonyl-Gruppe gebunden sind, insbesondere für Methylthiocarbonyl und Ethylthiocarbonyl.

($C_{1-18}$-Alkyl)NH steht erfindungsgemäß jeweils unabhängig voneinander für alle gesättigten und ungesättigten, linearen und verzweigten Alkylreste mit bis zu 18 C-Atomen, die über eine Hydrogenamino-Gruppe gebunden sind, wobei ($C_{1-6}$-Alkyl)NH bevorzugt ist. ($C_{1-6}$-Alkyl)NH steht erfindungsgemäß für alle gesättigten und ungesättigten, linearen und verzweigten Alkyl-Reste mit bis zu 6 C-Atomen, die über eine Hydrogenamino-Gruppe gebunden sind, insbesondere für $CH_3NH$ und $C_2H_5NH$.

Di-($C_{1-18}$-Alkyl)N steht erfindungsgemäß jeweils unabhängig voneinander für alle gesättigten und ungesättigten, linearen und verzweigten Alkylreste mit bis zu 18 C-Atomen, die über eine ($C_{1-18}$-Alkyl)amino-Gruppe gebunden sind, wobei Di-($C_{1-6}$-Alkyl)N bevorzugt ist. Die beiden Alkyl-Reste können hierbei gleich oder unterschiedlich voneinander sein. Di-($C_{1-6}$-Alkyl)N steht erfindungsgemäß für alle gesättigten und ungesättigten, linearen und verzweigten Alkylreste mit bis zu 6 C-Atomen, die über eine ($C_{1-6}$-Alkyl)amino-Gruppe gebunden sind, insbesondere für $(CH_3)_2N$ und $(C_2H_5)_2N$.

$C_{6-10}$-Aryl steht erfindungsgemäß, insbesondere auch in $C_{6-10}$-Aryl-$C_{1-12}$-alkyl, $C_{6-10}$-Aryloxy, $C_{6-10}$-Arylamino, $C_{6-10}$-Arylsulfanyl, $C_{6-10}$-Arylsulfonyl, $C_{6-10}$-Arylsulfoxidyl, $C_{6-10}$-Arylcarbonyl, $C_{6-10}$-Arylcarbonyloxy, $C_{6-10}$-Aryloxycarbonyl, $C_{6-10}$-Arylaminocarbonyl und $C_{6-10}$-Arylsulfanylcarbonyl, vorzugsweise für Phenyl oder Naphthyl, besonders bevorzugt für Phenyl.

Heteroaryl steht erfindungsgemäß, insbesondere auch in Heteroaryl-$C_{1-12}$-alkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl und Heteroarylsulfanylcarbonyl, sofern nicht anders angegeben, für einen mindestens ein Heteroatom ausgewählt aus O, S und N enthaltenden aromatischen Rest mit 5 bis 10, vorzugsweise 5 oder 6, Ringgliedern, vorzugsweise ausgewählt aus Furanyl, Thienyl, Thiophenyl, Pyrrolyl, Isopyrrolyl, Pyrazolyl, Imidazolyl, Oxazolyl, Thiazolyl, Isothiazolyl, Pyridinyl, Pyridazinyl, Pyrimidinyl, Pyrazinyl, Triazinyl, Benzofuranyl, Benzothiophenyl, Indolyl, Chinolinyl, Isochinolinyl, Benzimidazolyl, Indazolyl, Pyridofuranyl und Pyridothienyl.

In $C_{6-10}$-Aryl-$C_{1-12}$-alkyl und Heteroarylalkyl kann der Alkyl-Rest gesättigt oder ungesättigt, verzweigt oder unverzweigt sein. Bevorzugte Reste sind Benzyl, Phenylethyl, Naphthylmethyl und Naphthylethyl.

"Amino" steht erfindungsgemäß für jede beliebige substituierte oder unsubstituierte Aminogruppe, insbesondere für $-NH_2$, $-NH(C_{1-18}\text{-Alkyl})$, $-N(C_{1-18}\text{-Alkyl})_2$, $-NH(C_{6-10}\text{-Aryl})$ oder $-N(C_{6-10}\text{-Aryl})_2$.

"Ammonium" steht erfindungsgemäß für jede beliebige substituierte oder unsubstituierte Ammoniumgruppe, insbesondere für $-NH_3^{(+)}$, $-NH_2(C_{1-18}\text{-Alkyll})$, $-NH(C_{1-18}\text{-Alkyl})_2^{(+)}$ oder $-N(C_{1-18}\text{-Alkyl})_3^{(+)}$,

"Sulfato" steht erfindungsgemäß insbesondere für $-O-S(O)_2-O-R$, "Sulfo" für $-S(O)_2-O-R$, "Amidosulfo" für $-O-S(O)_2-NR_2$, "Phosphato" für $-O-P(O)(OR)_2$, "Phosphono" für $-P(O)(OR)_2$ "Amidophosphono" für $-O-P(O)(NR_2)_2$ oder-$O-P(O)(OR)(NR_2)$ und "Amidocarbonyl" für $-C(O)-NR_2$, wobei R jeweils unabhängig voneinander für H, $M^{(+)}$, $C_{1-18}$-Alkyl, $C_{6-10}$-Aryl oder $C_{1-18}$-Alkyl-$C_{6-10}$-Aryl steht.

Bei dem erfindungsgemäßen Metall-Ligand-Komplex handelt es sich vorzugsweise um einen Komplex mit einem Metall ausgewählt aus Ag, Al, Ce, Co, Cu, Fe, Mo, Mn, Ni, Pb, Re, Ti, V und Zn in beliebigen Oxidationsstufen, wobei das Metall vorzugsweise ausgewählt ist aus Co(II), Co(III), Cu(I), Cu(II), Fe(II), Fe(III), Mn(II), Mn(III), Ni(II), Pb (II) und Zn(II), besonders bevorzugt aus Mn(II) und Mn(III).

Die Herstellung des Metall-Ligand-Komplexes kann in der Regel auf einfache Weise dadurch erfolgen, dass ein Metallsalz des entsprechenden Metalls mit dem entsprechenden Ligand in wässriger Umgebung vermischt wird. Durch Einstellen eines geeigneten Redoxpotentials kann die Entstehung einer gewünschten Oxidationsstufe begünstigt werden.

Zur Absättigung der nach Bindung an den Liganden noch freien Valenzen und/oder noch freien Ladung kommt grundsätzlich jedes beliebige Gegenion in Frage, insbesondere Acetat, Tetrafluoroborat, Fluorid, Bromid, Iodid oder Chlorid.

Weiterer Gegenstand der vorliegenden Erfindung ist ebenfalls die Verwendung erfindungsgemäßer Wasch- und Reinigungsmittel zur Reinigung textiler Flächengebilde sowie zur Reinigung harter Oberflächen.

Die Metall-Ligand-Komplexe werden im Folgenden auch "erfindungsgemäße Bleichkatalysatoren" genannt.

Die Metall-Ligand-Komplexe von Liganden der allgemeinen Formel (I), insbesondere der allgemeinen Formel (II), vorzugsweise der allgemeinen Formel (III), sind insbesondere

dadurch gekennzeichnet, dass das Übergangsmetallatom ausgewählt ist aus Ag, Ce, Co, Cu, Fe, Mo, Mn, Ni, Pb, Re, Ti und V in beliebigen Oxidationsstufen, insbesondere aus Co(II), Co(III), Cu(I), Cu(II), Fe(II), Fe(III), Mn(II), Mn(III), Ni (II) und Pb(II), besonders bevorzugt aus Mn(II) und Mn(III),

X für $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ oder $SiR^3R^4$ steht,

Y und Z unabhängig voneinander für OH, SH, $NH_2$, $NHR^5$ oder $NR^5R^6$ stehen,

A, B, C. D, $R^1$, $R^2$, $R^3$, $R^4$, $R^5$ und $R^6$ unabhängig voneinander für einen der zuvor genannten Reste stehen,

ferner dadurch gekennzeichnet, dass mindestens einer der Reste A, B, C, D, $R^1$, $R^2$, $R^3$, $R^4$, $R^5$ und $R^6$ für einen anderen Substituenten als Wasserstoff steht, wobei vorzugsweise mindestens einer der Reste A, B, C, D, $R^1$, $R^2$, $R^3$, $R^4$, $R^5$ und $R^6$ für einen Substituenten umfassend eine Gruppe ausgewählt aus Ammonium, Nitro, Sulfato, Sulfo, Amidosulfo, Hydroxycarbonyl, Alkoxycarbonyl, Aryloxycarbonyl, Amidocarbonyl, Phosphato, Phosphono, Amidophosphono, Hydroxy, Alkoxy, Amino, Polyoxyethylen und Halogen steht.

Metall-Ligand-Komplexe von Liganden der allgemeinen Formel (I), insbesondere der allgemeinen Formel (II), vorzugsweise der allgemeinen Formel (III), sind insbesondere
dadurch gekennzeichnet, dass das Übergangsmetallatom ausgewählt ist aus Ag, Al, Ce, Co, Cu, Fe, Mo, Mn, Ni, Pb, Re, Ti, U, V und Zn in beliebigen Oxidationsstufen, insbesondere aus Co(II), Co(III), Cu(I), Cu(II), Fe(II), Fe(III), Mn(II), Mn(III), Ni(II), Pb(II), U(IV) und Zn(II), besonders bevorzugt aus Mn(II) und Mn(III),
X für $PR^1$, $PR^1R^{2(+)}$, $P(O)R^1$, O, S, $BR^1$, $BR^1R^{2(-)}$, $CR^3R^4$ oder $SiR^3R^4$ steht,
Y und Z unabhängig voneinander für OH, SH, $NH_2$, $NHR^5$ oder $NR^5R^6$ stehen,
A, B, C, D, $R^1$, $R^2$, $R^3$, $R^4$, $R^5$ und $R^6$ unabhängig voneinander für einen der zuvor genannten Reste stehen, wobei vorzugsweise mindestens einer der Reste A, B, C, D, $R^1$, $R^2$, $R^3$, $R^4$, $R^5$ und $R^6$ für einen Substituenten umfassend eine Gruppe ausgewählt aus Ammonium, Nitro, Sulfato, Sulfo, Amidosulfo, Hydroxycarbonyl, Alkoxycarbonyl, Aryloxycarbonyl, Amidocarbonyl, Phosphato, Phosphono, Amidophosphono, Hydroxy, Alkoxy, Amino, Polyoxyethylen und Halogen steht.

Weiterer Gegenstand der vorliegenden Erfindung ist ebenso die Verwendung erfindungsgemäßer Liganden und/oder Metall-Ligand-Komplexe in Wasch- oder Reinigungsmitteln, insbesondere zur Reinigung textiler Flächengebilde sowie zur Reinigung harter Oberflächen.

Weiterer Gegenstand der vorliegenden Erfindung ist ebenso die Verwendung erfindungsgemäßer Liganden und/oder Metall-Ligand-Komplexe, insbesondere als Hilfsmittel, zur Reinigung textiler Flächengebilde sowie zur Reinigung harter Oberflächen.

Weiterer Gegenstand der vorliegenden Erfindung ist ebenso die Verwendung erfindungsgemäßer Liganden und/oder Metall-Ligand-Komplexe zum Bleichen von Zellstoff und/oder Roh-Baumwolle.

Bei den erfindungsgemäßen Wasch- und Reinigungsmitteln kann es sich um alle denkbaren Reinigungsmittelarten handeln, sowohl um Konzentrate als auch um unverdünnt anzuwendende Mittel, zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Im weiteren Sinne sind auch Sterilisations- und Desinfektionsmittel als Wasch- und Reinigungsmittel im erfindungsgemäßen Sinne anzusehen.

Ausführungsformen der vorliegenden Erfindung umfassen alle nach dem Stand der Technik etablierten und/ oder alle zweckmäßigen Darreichungsformen der erfindungsgemäßen Wasch- oder Reinigungsmittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

In einer bevorzugten Ausführungsform enthalten die erfindungsgemäßen Wasch- oder Reinigungsmittel die oben beschriebenen erfindungsgemäßen Bleichkatalysatoren in einer Menge von bis zu 5 Gew.-%, insbesondere von 0,001 Gew.-% bis 1 Gew.-% und besonders bevorzugt von 0,01 Gew.-% bis 0,5 Gew.-%, vor allem von 0,01 bis 0,25 Gew.-%, jeweils bezogen auf das Gesamtgewicht des Wasch- oder Reinigungsmittels.

Neben den erfindungsgemäßen Bleichkatalysatoren können gegebenenfalls zusätzlich auch andere Bleichkatalysatoren in den erfindungsgemäßen Mitteln enthalten sein. Bei diesen Stoffen kann es sich generell um jedes beliebige bleichverstärkende Übergangsmetallsalz beziehungsweise jeden beliebigen Übergangsmetallkomplex handeln. Als Übergangsmetalle kommen hierbei insbesondere Mn, Fe, Co, Ru, Mo, Ti, V oder Cu in unterschiedlichen Oxidationsstufen in Betracht. Als mögliche komplexierende Liganden kommen insbesondere, wie in der Literatur beschrieben, Guanidine, Aminophenole, Aminoxide, Salene, Saldimine, Lactame, monocyclische sowie querverbrückte polycyclische Polyazaalkane, Terpyridine, Dendrimere, Tetraamido-Liganden, Bis- und Tetrakis(pyridylmethyl)alkylamine, sekundäre Amine und Polyoxometallate in Betracht.

In einer bevorzugten Ausführungsform wird als zusätzlicher Bleichkatalysator ein Komplex des Mangans in der Oxidationsstufe II, III, IV oder V eingesetzt, der vorzugsweise einen oder mehrere makrocyclische Liganden mit den Donorfunktionen N, NR, PR, O und/oder S enthält. Vorzugsweise werden hierbei Liganden eingesetzt, die Stickstoff-Donorfunktionen aufweisen. Dabei ist es besonders bevorzugt, zusätzlich einen Bleichkatalysator in den erfindungsgemäßen Mitteln einzusetzen, der als makromolekularen Liganden 1,4,7-Trimethyl-1,4,7-triazacyclononan (Me-TACN), 1,4,7-Triazacyclononan (TACN), 1,5,9-Trimethyl-1,5,9-triazacyclododecan (Me-TACD), 2-Methyl-1,4,7-trimethyl-1,4,7-triazacyclononan (Me/Me-TACN) und/oder 2-Methyl-1,4,7-triazacyclononan (Me/TACN) enthält. Geeignete Mangankomplexe sind beispielsweise $[Mn^{III}_2(\mu\text{-}O)_1(\mu\text{-}OAc)_2(TACN)_2](ClO_4)_2$, $[Mn^{III}Mn^{IV}(\mu\text{-}O)_2(\mu\text{-}OAc)_1(TACN)_2](BPh_4)_2$, $[Mn^{IV}_4(\mu\text{-}O)_6(TACN)_4](ClO_4)_4$, $[Mn^{III}_2(\mu\text{-}O)_1(\mu\text{-}OA_C)_2(Me\text{-}TACN)_2](ClO_4)_2$, $[Mn^{III}Mn^{IV}(\mu\text{-}O)_1(\mu\text{-}OAc)_2(Me\text{-}TACN)_2](ClO_4)_3$, $[Mn^{IV}_2(\mu\text{-}O)_3(Me\text{-}TACN)_2](PF_6)_2$ und $[Mn^{IV}_2(\mu\text{-}O)_3(Me/Me\text{-}TACN)_2](PF_6)_2$ (OAc = $OC(O)CH_3$).

Auch der zusätzliche Bleichkatalysator ist, soweit eingesetzt, in den erfindungsgemäßen Mitteln vorzugsweise in einer Menge von bis zu 5 Gew.-%, insbesondere von 0,0025 Gew.-% bis 1 Gew.-% und besonders bevorzugt von 0,01 Gew.-% bis 0,25 Gew.-%, jeweils bezogen auf das Gesamtgewicht des Wasch- oder Reinigungsmittels, enthalten.

Des Weiteren sind in den erfindungsgemäßen Wasch- und Reinigungsmitteln vorzugsweise Bleichmittel enthalten, die vorzugsweise das Substrat für die erfindungsgemäßen Bleichkatalysatoren darstellen und/oder liefern. Unter einem Bleichmittel ist in diesem Sinne zum einen Wasserstoffperoxid selbst und zum anderen jede Verbindung, die in wässrigem Medium Wasserstoffperoxid liefert, zu verstehen. Unter den als Bleichmittel dienenden, in Wasser $H_2O_2$ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie $H_2O_2$ liefernde persaure Salze oder Persäuren, wie Persulfate beziehungsweise Perschwefelsäure. Brauchbar ist auch das Harnstoffperoxohydrat Percarbamid, das durch die Formel $H_2N\text{-}CO\text{-}NH_2 \cdot H_2O_2$ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-$\alpha$-Naphthoesäure und Magnesium-monoperphthalat, (b) die aliphatischen oder substituiert aliphatischen Peroxysäuren, wie Peroxylaurinsäure, Peroxystearinsäure, $\varepsilon$-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und (c) aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure).

Als Bleichmittel können auch Chlor oder Brom freisetzende Substanzen eingesetzt werden. Unter den geeigneten Chlor oder Brom freisetzenden Materialien kommen beispielsweise heterozyklische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

In einer besonderen erfindungsgemäßen Ausführungsform wird auf den Einsatz von Wasserstoffperoxid liefernden Substanzen verzichtet und als Bleichmittel wird stattdessen Sauerstoff eingesetzt, wobei es sich bei dem Sauerstoff um Luftsauerstoff handeln kann oder um Sauerstoff, der aus einem Sauerstoff liefernden Mittel freigesetzt wird.

Erfindungsgemäß werden Wasch- oder Reinigungsmittel, insbesondere maschinelle Geschirrspülmittel, bevorzugt, die bis zu 45 Gew.-%, insbesondere 1 bis 35 Gew.-%, vorzugsweise 2,5 bis 30 Gew.-%, besonders bevorzugt 3,5 bis 20 Gew.-% und insbesondere 5 bis 15 Gew.-% Bleichmittel, vorzugsweise Natriumpercarbonat, enthalten.

Der Aktivsauerstoffgehalt der Wasch- oder Reinigungsmittel, insbesondere der maschinellen Geschirrspülmittel, beträgt, jeweils bezogen auf das Gesamtgewicht des Mittels, vorzugsweise zwischen 0,4 und 10 Gew.-%, besonders bevorzugt zwischen 0,5 und 8 Gew.-% und insbesondere zwischen 0,6 und 5 Gew.-%. Besonders bevorzugte Mittel weisen einen Aktivsauerstoffgehalt oberhalb 0,3 Gew.-%, bevorzugt oberhalb 0,7 Gew.-%, besonders bevorzugt oberhalb 0,8 Gew.-% und insbesondere oberhalb 1,0 Gew.-% auf.

Alternativ zu als auch gleichzeitig mit den Bleichmitteln können zum Bereitstellen des Wasserstoffperoxids auch Enzyme eingesetzt werden, die ausgehend von anderen Substraten dazu in der Lage sind, Wasserstoffperoxid in situ zu erzeugen. Es handelt sich hierbei um Oxidoreduktasen, die Elektronen von - in der Regel - einem organischen Substrat, etwa der Glucose, auf Sauerstoff als Elektronenakzeptor transferieren können und so die Ausbildung des gewünschten Wasserstoffperoxids in situ ermöglichen. Die Oxidoreduktase kann hierbei zusammen mit dem entsprechenden organischen Substrat eingesetzt werden. Da die zu behandelnden Anschmutzungen jedoch bereits das erforderliche Substrat enthalten können, kann der Einsatz der Oxidoreduktasen gegebenenfalls auch ohne Zusatz des entsprechenden Substrats erfolgen.

Bei der Wasserstoffperoxid erzeugenden Oxidoreduktase handelt es sich vorzugsweise um eine Oxidoreduktase, die Wasserstoffperoxid produziert, indem sie Sauerstoff als Elektronenakzeptor verwendet. Hierbei kommen insbesondere Oxidoreduktasen der EC-Klassen E.C. 1.1.3 (CH-OH als Elektronendonor), E.C. 1.2.3 (Aldehyd oder Oxo-Gruppe als Elektronendonor), E.C. 1.4.3 ($CH\text{-}NH_2$ als Donor), E.C. 1.7.3 (N-haltige Gruppe als Donor) und E.C. 1.8.3 (S-haltige Gruppe als Donor) in Betracht, wobei Enzyme der EC-Klasse E.C. 1.1.3 bevorzugt sind.

Bevorzugte Enzyme sind insbesondere ausgewählt aus der Gruppe bestehend aus Malat-Oxidase (EC 1.1.3.3), Glucose-Oxidase (EC 1.1.3.4), Hexose-Oxidase (EC 1.1.3.5), Cholesterin-Oxidase (EC 1.1.3.6), Galactose-Oxidase (EC 1.1.3.9), Pyranose-Oxidase (EC 1.1.3.10), Alkohol-Oxidase (EC 1.1.3.13), Cholin-Oxidase (EC 1.1.3.17, siehe

insbesondere WO 04/58955), Oxidasen für langkettige Alkohole (EC 1.1.3.20), Glycerin-3-phosphat-Oxidase (EC 1.1.3.21), Cellobiose-Oxidase (EC 1.1.3.25), Nucleosid-Oxidase (EC 1.1.3.39), D-Mannitol-Oxidase (EC 1.1.3.40), Xylitol-Oxidase (EC 1.1.3.41), Aldehyd-Oxidase (EC 1.2.3.1), Pyruvat-Oxidase (EC 1.2.3.3), Oxalat-Oxidase (EC 1.2.3.4), Glyoxylat-Oxidase (EC 1.2.3.5), Indol-3-acetaldehyd-Oxidase (EC 1.2.3.7), Pyridoxal-Oxidase (EC 1.2.3.8), Arylaldehyd-Oxidase (EC 1.2.3.9), Retinal-Oxidase (EC 1.2.3.11), L-Aminosäure-Oxidase (EC 1.4.3.2), Amin-Oxidase (EC 1.4.3.4, EC 1.4.3.6), L-Glutamat-Oxidase (EC 1.4.3.11), L-Lysin-Oxidase (EC 1.4.3.14), L-Aspartat-Oxidase (EC 1.4.3.16), Tryptophan-alpha,beta-Oxidase (EC 1.4.3.17), Glycin-Oxidase EC 1.4.3.19), Harnstoff-Oxidase (EC 1.7.3.3), Thiol-Oxidase (EC 1.8.3.2), Glutathion-Oxidase (EC 1.8.3.3), Sorbitol-Oxidase sowie aus Enzymen wie etwa in DE102005053529 beschrieben.

Bei der Wasserstoffperoxid produzierenden Oxidoreduktase handelt es sich in einer bevorzugten Ausführungsform um eine, die einen Zucker als Elektronendonor verwendet. Die Wasserstoffperoxid produzierende und Zucker oxidierende Oxidoreduktase ist erfindungsgemäß vorzugsweise ausgewählt aus Glucose-Oxidase (EC 1.1.3.4), Hexose-Oxidase (EC 1.1.3.5), Galactose-Oxidase (EC 1.1.3.9) und Pyranose-Oxidase (EC 1.1.3.10). Besonders bevorzugt ist erfindungsgemäß die Glucose-Oxidase (EC 1.1.3.4).

Vorteilhafterweise werden bei Verwendung einer Wasserstoffperoxid erzeugenden Oxidoreduktase zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die Wasserstoffperoxid produzierende Oxidoreduktase wird in den erfindungsgemäßen Wasch-und Reinigungsmitteln, falls sie verwendet werd, vorzugsweise in einer solchen Menge eingesetzt, dass das gesamte Mittel eine auf die Oxidoreduktase bezogene Enzym-Aktivität von 30 U/g bis 20.000 U/g, insbesondere von 60 U/g bis 15.000 U/g aufweist. Die Einheit 1 U (Unit) entspricht hierbei der Aktivität derjenigen Enzymmenge, die 1 μmol ihres Substrats bei pH 7 und 25 °C in einer Minute umsetzt.

Das gegebenenfalls bei Verwendung einer solchen Wasserstoffperoxid produzierenden Oxidoreduktase einzusetzende Substrat ergibt sich in der Regel unmittelbar aus der Bezeichnung der jeweiligen Oxidoreduktase.

Erfindungsgemäße Mittel können gegebenenfalls auch Bleichaktivatoren als zusätzlichen Bleichhilfsstoff enthalten. Hinsichtlich erfindungsgemäß vorzugsweise einsetzbarer Bleichaktivatoren und deren bevorzugten Einsatzmengen wird auf die Offenlegungsschrift WO2008/135337 verwiesen.

Neben einem erfindungsgemäßen Bleichkatalysator und den zuvor genannten Bleichmitteln und optional enthaltenen weiteren Bleichhilfsstoffen enthält ein erfindungsgemäßes Wasch- oder Reinigungsmittel gegebenenfalls weitere Inhaltsstoffe wie weitere Enzyme, Enzymstabilisatoren, Tenside, insbesondere nichtionische, anionische, kationische und/oder amphotere Tenside, Gerüststoffe (Builder, Cobuilder), Polymere, Lösungsmittel, Verdicker, Sequestrierungsmittel, Elektrolyte, gasentwickelnde Brausesysteme, optische Aufheller, Vergrauungsinhibitoren, Glaskorrosionsinhibitoren, Korrosionsinhibitoren, Farbübertragungsinhibitoren, Schauminhibitoren, Desintegrationshilfsstoffe, Abrasivstoffe, Farbstoffe, Duftstoffe, mikrobielle Wirkstoffe, UV-Absorbenzien, synthetische Knitterschutzmittel, Antistatika, sogenannte Soil-Release-Wirkstoffe bzw. Soil-Repellents, Treibmittel sowie gegebenenfalls weitere übliche Inhaltsstoffe.

Hinsichtlich erfindungsgemäß vorzugsweise einsetzbaren weiteren Enzymen, Enzymstabilisatoren, Tensiden, Gerüststoffen, Polymeren, Lösungsmitteln, Verdickern, Sequestrierungsmitteln, Elektrolyten, Acidifizierungsmitteln, optischen Aufhellern, Vergrauungsinhibitoren, Glaskorrosionsinhibitoren, Korrosionsinhibitoren, Farbobertragungsinhibitoren, Schauminhibitoren, Desintegrationshilfsstoffen, Abrasivstoffen, Farbstoffen, Duftstoffen, mikrobiellen Wirkstoffen, UV-Absorbenzien, Knitterschutzmitteln, Antistatika, Soil-Release-Wirkstoffen und Treibmitteln sowie deren bevorzugten Einsatzmengen wird auf die Offenlegungsschrift WO2008/135337 verwiesen.

Als besonders vorteilhafte Bleichkatalysatorgranulate haben sich solche herausgestellt, die bezogen auf das Gesamtgewicht des Granulats

a) 0,1 bis 30 Gew.-% eines erfindungsgemäßen Bleichkatalysators sowie gegebenenfalls weiteren Bleichkatalysator,
b) 10 bis 99 Gew.-% eines Trägermaterials, sowie
c) 0,1 bis 5 Gew.-% eines Bindemittels aus der Gruppe der organischen Polymere enthalten.

Der gegebenenfalls einzusetzende weitere Bleichkatalysator gemäß a) ist hierbei vorzugsweise ausgewählt aus den bereits zuvor genannten weiteren Bleichkatalysatoren.

Als Trägermaterial b) eignen sich grundsätzlich alle in Wasch- und Reinigungsmitteln einsetzbaren mit den übrigen Inhaltsstoffen kompatiblen Substanzen oder Substanzgemische, insbesondere die bereits zuvor aufgezählten Gerüststoffe, vor allem die Carbonate, einschließlich der Hydrogencarbonate, die Sulfate, die Chloride, die Silikate und die Phosphate. Als Tragermaterial eignen sich hierbei Insbesondere Alkalimetallcarbonate, Alkalimetallhydrogencarbonate, Alkalimetallsesquicarbonate, Alkalisilikate, Alkalimetasilikate, Alkaliphosphate und Mischungen dieser Stoffe, wobei im Sinne dieser Erfindung bevorzugt die Alkalicarbonate, insbesondere Natriumcarbonat, Natriumhydrogencarbonat

oder Natriumsesquicarbonat, und/oder Alkaliphosphate eingesetzt werden. In einer besonders bevorzugten Ausführungsform wird als Trägermaterial das Pentanatriumtriphosphat, $Na_5P_3O_{10}$ (Natriumtripolyphosphat) oder das entsprechende Kaliumsalz Pentakaliumtriphosphat, $K_5P_3O_{10}$ (Kaliumtripolyphosphat) eingesetzt.

Der Gewichtsanteil des Trägermaterials b) am Gesamtgewicht der Bleichkatalysatorgranulate kann in eingangs angegebenen Grenzen variiert werden, wobei sich hinsichtlich der Verarbeitbarkeit und der tatsächlichen Bleichleistung nach der Konfektionierung mit weiteren wasch- und reinigungsaktiven Inhaltsstoffen insbesondere Gewichtsanteile oberhalb 20 Gew.-%, vorzugsweise oberhalb 40 Gew.-% und insbesondere oberhalb 60 Gew.-% als vorteilhaft erwiesen haben. Folglich werden im Rahmen der vorliegenden Anmeldung Bleichkatalysatorgranulate bevorzugt, bei denen der Gewichtsanteil des Trägermaterials b) am Gesamtgewicht des Granulats 20 bis 99 Gew.-%, vorzugsweise zwischen 40 und 95 Gew.-% und insbesondere zwischen 60 und 90 Gew.-% beträgt.

Als dritten Inhaltsstoff enthalten die erfindungsgemäßen Bleichaktivatorgranulate ein Bindemittel c) aus der Gruppe der organischen Polymere. Die Polymere können nichtionischer, anionscher, kationischer oder amphoterer Natur sein. Natürliche Polymere und modifizierte Polymere natürlichen Ursprungs sind ebenso einsetzbar wie synthetische Polymere.

Zur Gruppe der mit besonderem Vorzug als Bindemittel c) eingesetzten nichtionischen Polymere zählen Polyvinylalkohole, acetalisierte Polyvinylalkohole, Polyvinylpyrrolidone und Polyalkylenglykole, insbesondere Polyethylenoxide. Bevorzugte Polyvinylalkohole und acetalisierte Polyvinylalkohole weisen Molekulargewicht im Bereich von 10.000 bis 100.000 $gmol^{-1}$, vorzugsweise von 11.000 bis 90.000 $gmol^{-1}$, besonders bevorzugt von 12.000 bis 80.000 $gmol^{-1}$ und insbesondere von 13.000 bis 70.000 $gmol^{-1}$ auf. Bevorzugte Polyethylenoxide haben Molmassen im Bereich von ca. 200 bis 5.000.000 g/mol, entsprechend Polymerisationsgraden n von ca. 5 bis >100.000.

Zu der Gruppe der mit besonderem Vorzug als Bindemittel c) eingesetzten anionischen Polymere gehören insbesondere homo- oder copolymere Polycarboxylate, Polyacrylsäuren und Polymethacrylsäuren, insbesondere solche, die bereits zuvor als für Wasch- und Reinigungsmittel brauchbare organische Gerüstsubstanzen genannt wurden, sowie Sulfonsäuregruppen-haltige Polymere, insbesondere solche, die bereits zuvor als brauchbare Enthärter genannt wurden.

Hinsichtlich der Gruppe der mit besonderem Vorzug als Bindemittel c) eingesetzten kationischen und amphoteren Polymere wird auf die bereits zuvor als wasch- und reinigungsaktive Polymere aufgezählten Polymere verwiesen.

In erfindungsgemäß bevorzugten Bleichkatalysatorgranulaten beträgt der Gewichtsanteil des Bindemittels c) am Gesamtgewicht des Granulats zwischen 0,2 und 4,5 Gew.-%, bevorzugt zwischen 0,5 und 4,0 Gew.-% und insbesondere zwischen 1,0 und 4,0 Gew.-%.

Die Bleichkatalysatorgranulate besitzen vorzugsweise eine mittlere Teilchengröße zwischen 0,1 und 1,0 mm, besonders bevorzugt zwischen 0,2 und 0,8 mm und insbesondere zwischen 0,3 und 0,7 mm, wobei der Gewichtsanteil der Teilchen mit einer Teilchengröße unterhalb 0,1 mm vorzugsweise mindestens 4 Gew.-%, besonders bevorzugt mindestens 6 Gew.-% und insbesondere mindestens 8 Gew.-%, jedoch gleichzeitig vorzugsweise maximal 80 Gew.-%, besonders bevorzugt maximal 60 Gew.-% und insbesondere maximal 40 Gew.-% beträgt, und der Gewichtsanteil der Teilchen mit einer Teilchengröße zwischen 0,2 und 0,8 mm vorzugsweise zwischen 30 und 70 Gew.-%, besonders bevorzugt zwischen 45 und 65 Gew.-% und insbesondere zwischen 40 und 60 Gew.-% beträgt

Außer dem Bleichkatalysator können auch Enzyme oder andere Inhaltsstoffe, insbesondere sensitive, auf die zuvor beschriebene Art und Weise konfektioniert werden.

Einen eigenen Erfindungsgegenstand stellen Verfahren zur Reinigung von Textilien oder von harten Oberflächen dar, bei denen wenigstens in einem der Verfahrensschritte ein erfindungsgemäßer Bleichkatalysator verwendet wird.

Hierunter fallen sowohl manuelle als auch maschinelle Verfahren. Ausführungsformen stellen beispielsweise die Handwäsche, die manuelle Entfernung von Flecken von Textilien oder von harten Oberflächen oder die Verwendung im Zusammenhang mit einem maschinellen Verfahren dar, wobei maschinelle Verfahren, insbesondere zur Reinigung von Textilien, aufgrund ihrer präziseren Steuerbarkeit, was beispielsweise die eingesetzten Mengen und Einwirkzeiten angeht, bevorzugt sind. Entsprechend bevorzugt gelten für diese Verwendungen die oben angeführten Konzentrationsbereiche.

Die Reinigung der textilen Flächengebilde erfolgt vorzugsweise bei Temperaturen von 20 - 95°C, in einer bevorzugten Ausführungsform bei Temperaturen von 20 - 60°C, insbesondere bei Temperaturen von 20 - 40°C, sowie vorzugsweise bei einem pH-Wert von 5-12, insbesondere von 8-11.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird. Das gleiche gilt für Verfahren zur Reinigung von allen anderen Materialien als Textilien, welche unter dem Begriff "harte Oberflächen" zusammengefasst werden. Alle denkbaren Wasch- oder Reinigungsverfahren können in wenigstens einem der Verfahrensschritte um einen erfindungsgemäßen Bleichkatalysator bereichert werden, und stellen dann Ausführungsformen der vorliegenden Erfindung dar.

In einer bevorzugten Ausführungsform dieser Verwendung werden die erfindungsgemäßen Bleichkatalysatoren hierbei im Rahmen einer der oben ausgeführten Rezepturen für erfindungsgemäße Mittel, vorzugsweise Wasch- beziehungsweise Reinigungsmittel, bereitgestellt.

Ein weiterer Gegenstand der vorliegenden Erfindung ist auch ein Erzeugnis, enthaltend eine erfindungsgemäße Zusammensetzung bzw. ein erfindungsgemäßes Wasch- oder Reinigungsmittel, insbesondere einen erfindungsgemäßen Reiniger für harte Oberflächen, und einen Sprühspender. Bei dem Erzeugnis kann es sich hierbei sowohl um ein Einkammer- als auch um ein Mehrkammerbehältnis, insbesondere ein Zweikammerbehältnis handeln. Bevorzugt ist der Sprühspender hierbei ein manuell aktivierter Sprühspender, insbesondere ausgewählt aus der Gruppe umfassend Aerosolsprühspender (Druckgasbehälter; auch u.a. als Spraydose bezeichnet), selbst Druck aufbauende Sprühspender, Pumpsprühspender und Triggersprühspender, insbesondere Pumpsprühspender und Triggersprühspender mit einem Behälter aus transparentem Polyethylen oder Polyethylenterephthalat. Sprühspender werden ausführlicher in der WO 96/04940 (Procter & Gamble) und den darin zu Sprühspendern zitierten US-Patenten, auf die in dieser Hinsicht sämtlich Bezug genommen und deren Inhalt hiermit in diese Anmeldung aufgenommen wird, beschrieben. Triggersprühspender und Pumpzerstäuber besitzen gegenüber Druckgasbehältern den Vorteil, daß kein Treibmittel eingesetzt werden muß. Durch geeignete, partikelgängige Aufsätze, Düsen etc. (sog. "nozzle-Ventile") auf dem Sprühspender kann gegebenenfalls enthaltenes Enzym in dieser Ausführungsform optional auch in auf Partikeln immobilisierter Form dem Mittel beigefügt werden und so als Reinigungsschaum dosiert werden.

Erfindungsgemäß besonders bevorzugte maschinelle Geschirrspülmittel umfassen

- 5 bis 70 Gew.-%, vorzugsweise 10 bis 60 Gew.-% und insbesondere 20 bis 50 Gew.-% Gerüstoff(e), mit Ausnahme wasch- und reinigungsaktiver Polymere;
- 2 bis 28 Gew.-%, vorzugsweise 4 bis 20 Gew.-% und insbesondere 6 bis 15 Gew.-% wasch-und reinigungsaktive Polymere;
- 0,5 bis 10 Gew.-%, vorzugsweise 1 bis 8 Gew.-% und insbesondere 2 bis 6 Gew.-% Tensid(e), vorzugsweise nichtionische(s) und/oder amphotere(s) Tensid(e);
- 0,5 bis 8 Gew.-%, vorzugsweise 1 bis 7 Gew.-% und insbesondere 2 bis 6 Gew.-% Enzym(e);
- 2 bis 20 Gew.-%, vorzugsweise 4 bis 15 Gew.-% und insbesondere 6 bis 12 Gew.-% Bleichmittel;
- 0,01 bis 5 Gew.-%, vorzugsweise 0,02 bis 4 Gew.-% und insbesondere 0,05 bis 3 Gew.-% erfindungsgemäße Bleichkatalysatoren; sowie gegebenenfalls
- 0,01 bis 5 Gew.-%, vorzugsweise 0,02 bis 4 Gew.-% und insbesondere 0,05 bis 3 Gew.-% weitere Bleichkatalysatoren.

Ganz besonders bevorzugte maschinelle Geschirrspülmittel umfassen

- 5 bis 70 Gew.-%, vorzugsweise 10 bis 60 Gew.-% und insbesondere 20 bis 50 Gew.-% Phosphate;
- 2 bis 28 Gew.-%, vorzugsweise 4 bis 20 Gew.-% und insbesondere 6 bis 15 Gew.-% wasch-und reinigungsaktive Polymere;
- 0,5 bis 10 Gew.-%, vorzugsweise 1 bis 8 Gew.-% und insbesondere 2 bis 6 Gew.-% nichtionische(s) Tensid(e);
- 0,5 bis 8 Gew.-%, vorzugsweise 1, bis 7 Gew.-% und insbesondere 2 bis 6 Gew.-% Enzym(e) ausgewählt aus Amylasen, Proteasen und Amadoriasen;
- 2 bis 20 Gew.-%, vorzugsweise 4 bis 15 Gew.-% und insbesondere 6 bis 12 Gew.-% Percarbonat;
- 0,01 bis 5 Gew.-%, vorzugsweise 0,02 bis 4 Gew.-% und insbesondere 0,05 bis 3 Gew.-% erfindungsgemäße Bleichkatalysatoren; sowie gegebenenfalls
- 0,01 bis 5 Gew.-%, vorzugsweise 0,02 bis 4 Gew.-% und insbesondere 0,05 bis 3 Gew.-% weitere Bleichkatalysatoren.

Die Konfektionierung erfindungsgemäßer maschineller Geschirrspülmittel kann in unterschiedlicher Weise erfolgen. Die erfindungsgemäßen Mittel können in fester oder flüssiger sowie als Kombination fester und flüssiger Angebotsformen vorliegen.

Als feste Angebotsformen eignen sich insbesondere Pulver, Granulate, Extrudate oder Kompaktate, insbesondere Tabletten. Die flüssigen Angebotsformen auf Basis von Wasser und/oder organischen Lösungsmitteln können verdickt, in Form von Gelen vorliegen.

Erfindungsgemäße Mittel können in Form einphasiger oder mehrphasiger Produkte konfektioniert werden. Bevorzugt werden insbesondere maschinelle Geschirrspülmittel mit einer, zwei, drei oder vier Phasen. Maschinelle Geschirrspülmittel, dadurch gekennzeichnet, dass es in Form einer vorgefertigten Dosiereinheit mit zwei oder mehr Phasen vorliegt, werden besonders bevorzugt.

Die einzelnen Phasen mehrphasiger Mittel können die gleiche oder unterschiedliche Aggregatzustände aufweisen. Bevorzugt werden insbesondere maschinelle Geschirrspülmittel, die mindestens zwei unterschiedliche feste

Phasen und/oder mindestens zwei flüssige Phasen und/oder mindestens eine feste und mindestens eine feste Phase aufweisen.

Erfindungsgemäße maschinelle Geschirrspülmittel werden vorzugsweise zu Dosiereinheiten vorkonfektioniert. Diese Dosiereinheiten umfassen vorzugsweise die für einen Reinigungsgang notwendige Menge an wasch- oder reinigungsaktiven Substanzen. Bevorzugte Dosiereinheiten weisen ein Gewicht zwischen 12 und 30 g, bevorzugt zwischen 14 und 26 g und insbesondere zwischen 16 und 22 g auf.

Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Geschirrspülmaschine gewährleistet ist. Das Volumen der Dosiereinheit beträgt daher bevorzugt zwischen 10 und 35 ml, vorzugsweise zwischen 12 und 30 ml und insbesondere zwischen 15 und 25 ml.

Die erfindungsgemäßen maschinellen Geschirrspülmittel, insbesondere die vorgefertigten Dosiereinheiten weisen mit besonderem Vorzug eine wasserlösliche Umhüllung auf.

Die nachfolgenden Beispiele erläutern die Erfindung weiter, ohne sie darauf zu beschränken.

Ausführungsbeispiele

Beispiel 1: Darstellung der verwendeten bisq-Komplexe:

$[Mn_3(\textit{bisq})_2(L)_2(OAc)_2]$

$$3\ C_{19}H_{15}N_3O_2 + 2\ Mn(OAc)_2{*}4\ H_2O \rightarrow [Mn_3(\textit{N-Me-bisq})_2(L)_2(OAc)_2]$$

$$3\ C_{22}H_{21}N_3O_2 + 2\ Mn(OAc)_2{*}4\ H_2O \rightarrow [Mn_3(\textit{N-nBu-bisq})_2(L)_2(OAc)_2]$$

Die Darstellung der Komplexe gelingt durch Umsetzung des entsprechenden bisq-Liganden mit 1,5 Äquivalenten Mangan(II)acetat-tetrahydrat in Methanol.

Dazu werden jeweils 3 mmol des Liganden (Fluka) eingewogen, mit 30 ml Methanol oder Ethanol versetzt und zum Rückfluss erhitzt. Im Anschluss erfolgt die Zugabe von 1 mmol $Mn(OAC)_2$ als Feststoff oder methanolische Lösung, wobei sich nahezu sofort eine Suspension eines orangen Feststoffs in gelblicher Lösung bildet. Der Komplex kann nach Abkühlen auf Raumtemperatur quantitativ als orangener, feinkristalliner Feststoff abgetrennt werden.

$[Mn_3(\textit{N-nBu-bisq})_2(L)_2(OAC)_2]$:

IR (KBr): 3039, 2964, 2799, 1616, 1589, 1559, 1497, 1469, 1441, 1386, 1373, 1332, 1288, 1214, 1132, 1288, 1214, 1132, 1100, 1046, 858, 824, 794, 744, 661, 592, 540, 514, 430 $cm^{-1}$.

Vgl. Kristallstuktur: JDK51

$[Mn(\textit{bisq})(H_2O)]_2$

$$(C_{19}H_{13}N_3O_2)(NH_4^+)_2 + Mn(OAc)_2{*}4\ H_2O \rightarrow [Mn\ (\textit{N-Me-bisq})(L)]$$

$$(C_{22}H_{19}N_3O_2)(NH_4^+)_2 + Mn(OAC)_2{*}4\ H_2O \rightarrow [Mn(\textit{N-nBu-bisq})(L)]$$

Die Darstellung der Komplexe gelingt durch Umsetzung des entsprechenden *bisq*-Liganden mit 1 Äquivalent Mangan(II)acetat-tetrahydrat in ammoniakalischem Milieu.

Es wird jeweils 1 mmol des Liganden (Fluka) eingewogen, mit 30 ml Ethanol sowie 10 Tropfen konzentrierten Ammoniak versetzt und zum Rückfluss erhitzt. Im Anschluss erfolgt die Zugabe von 1 mmol $Mn(OAc)_2$ als wässrige Lösung. Der Komplex kann nach Abkühlen auf Raumtemperatur quantitativ als orangener, kristalliner Feststoff abgetrennt werden.

[Mn(*NnBu-bisq*)($H_2O$)]$_2$:

IR (KBr): 2928, 2858, 1640, 1613, 1586, 1555, 1495, 1443, 1383, 1333, 1301, 1199, 1154, 1130, 1097, 1025, 997, 965, 860, 816, 787, 749, 733, 706, 664, 645, 590, 560, 512 $cm^{-1}$.

Beispiel 2: Test auf Schädigung und Primärwaschleistung im miniaturisierten Waschtest des Mn-N-n-Bu-bisq-Komlexes

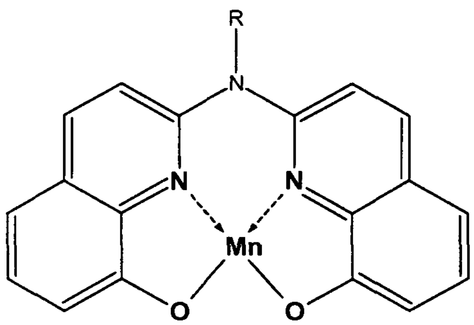

Mn-bisq-Komplexe mit R = Wasserstoff (bisq), n-Butyl (N-n-Bu-bisq) oder R = Methyl (N-n-Me-bisq)

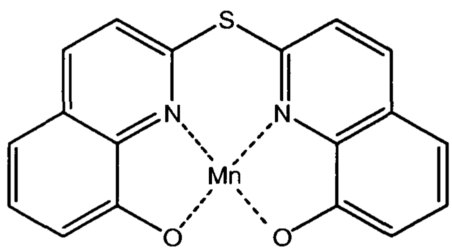

Mn-S-bisq-Komplex

Primärwaschkraft und Naßreißkraftverlust wurden in einem miniaturisierten Waschtest getestet. Es wurde mit einer kompletten flüssigen Waschmittelrezeptur gearbeitet. Die pH-Werte wurden in der Flüssigrezeptur mittels NaOH so eingestellt, dass bei einer Dosierung von 4,4 g/l und nach Zugabe der sonstigen Additive der jeweilige pH-Wert in der Waschformulierung vorlag. In Wasser mit 16°dH wurde 4,4 g/l Flüssigwaschmittel, 0, 35 g/l $H_2O_2$ und 3,6 mg/l Mn-N-nBu-bisq zugegeben. Die Kavitäten des MLA wurden mit jeweils 10 ml der Waschlauge gefüllt.

Für die Primärwaschleistung wurde ein Baumwollsubstrat mit der jeweiligen Anschmutzung im Probenbehälter eingespannt und der Behälter 1 h bei der gegebenen Temperatur in der Mikrowelle gedreht, so dass die Flüssigkeit in den Kavitäten mit der Baumwolle ständig in Kontakt kommt. Das behandelte Stoffsubstrat wurde unter fließendem lauwarmem Wasser ausgewaschen und anschließend getrocknet und Farbvermessen.

Für die Naßreißkraftverlust wurde in jede Kavität des Probenbehälters einen Baumwollstreifen mit definierter Breite (Fadenanzahl) hineingelegt und der Behälter 1 h bei 60°C in der Mikrowelle gedreht. Diese Behandlung wurde 20 Mal wiederholt. Die Streifen wurden getrocknet und in eine Netzlösung eingetaucht bevor sie mittels einer Zugprüfmaschine mit konstanter Prüfgeschwindigkeit zerrissen wurden. Die Zerreißkraft der behandelten Baumwolle wurde mit der Zerreißkraft der unbehandelten Baumwolle verglichen und die Naßreißkraftverlust in % berechnet.

Es wurden für die Primärwaschkraft und die Naßreißkraftverlust 5fach-Bestimmungen durchgeführt

Tabelle 1: Versuchsergebnisse zu Mn-N-nBu-bisq

| pH | Primärwaschkraft [Y-Wert] BC1, T= 30°C | Primärwaschkraft [Y-Wert] BC3, T = 30°C | Primärwaschkraft [Y-Wert] BC1, T = 60°C | Naßreißkraftverlust [%] |
|---|---|---|---|---|
| 7 | 48,1 | 47 | 49,2 | 7 |
| 8 | 47,9 | 46,9 | 50,8 | 5 |
| 9 | 48,9 | 46,8 | 52 | 5 |

(fortgesetzt)

| pH | Primärwaschkraft [Y-Wert] BC1, T= 30°C | Primärwaschkraft [Y-Wert] BC3, T = 30°C | Primärwaschkraft [Y-Wert] BC1, T = 60°C | Naßreißkraftverlust [%] |
|---|---|---|---|---|
| 10 | 49.6 | 48,1 | 54,9 | 5 |
| 11 | 51,4 | 50,2 | 58,6 | 10 |

Zum Vergleich sind im Folgenden die Ergebnisse für den Bleichkatalysator Mn-TACN dargestellt.

Tabelle 2: Versuchsergebnisse zu MnTACN

| pH | Primärwaschkraft [Y-Wert] BC1. T = 30°C | Primärwaschkraft [Y-Wert] BC3, T = 30°C | Primärwaschkraft [Y-Wert] BC1, T = 60°C | Naßreißkraftverlust [%] |
|---|---|---|---|---|
| 7 | 50,4 | 48,4 | n.d. | 20 |
| 8 | 52,0 | 51,3 | n.d. | 31 |
| 9 | 53,8 | 54,4 | n.d. | 33 |
| 10 | 57,0 | 56,2 | n.d. | 57 |
| 11 | 60,3 | 58,5 | n.d. | 83 |

Es ist zu erkennen, dass die Primärwaschkraft des Mn-N-nBu-bisq-Komplexes bei 30°C bei den verschiedenen getesteten pH-Werten zwar insgesamt schwächer ist als die des Mn-TACN, aber dennoch einen akzeptablen Wert aufweist. Der große Vorteil gegenüber Mn-TACN ist, dass der Naßreißkraftverlust bei Verwendung von Mn-timp deutlich geringer ist als bei Verwendung von Mn-TACN, so dass insgesamt der Quotient zwischen Waschkraft und Schädigung beim Mn-timp deutlich besser ist als beim Mn-TACN.

Beispiel 3: Waschtests in Modellwaschanlage

Der Waschtest wird in einer temperierbaren Multirührapparatur durchgeführt.

Als Testgefäße dienen 1l Bechergläser, in denen eine Vorrichtung zum mechanischen Rühren der Waschflotte existiert. Die Rührmechanik ist so ausgelegt, dass zum einen alle Bechergläser mit der selben Geschwindigkeit gerührt werden und zum anderen die Rührrichtung periodisch wechselt. Die Beladung der Waschkammern erfolgt mit ca. 16 g Ballastwäsche und ca. 6 g angeschmutztem Gewebe (die Gewebestücke werden in quadratische Form von ca. 6 cm Kantenlänge geschnitten und bestehen aus Baumwolle). Alle Testgewebe werden von der Firma CFT B.V. (Niederlande) hergestellt.

Bei dem angeschmutzten Gewebe handelt es sich um folgende bleichrelevante Testsubstrate:

| | |
|---|---|
| CS-103 | Rotwein |
| CS-3 | Rotwein gealtert |
| BC-1 | Tee |
| BC-3 | Tee |
| CS-15 | Heidelbeersaft |

Aus diesen 5 Testgeweben wird ein Satz von acht angeschmutzten Gewebestücken für die Tests zusammengestellt. Dies bedeutet, dass drei Anschmutzungen doppelt im Test vertreten sind.

Um die Bleichleistung zu ermitteln wird der Tristimulusvalue Y (Helligkeitswert) der gebleichten Gewebe ermittelt und mit den Referenzproben verglichen. Der Tristimulusvalue Y wird aus dem gemessenen L-Wert über folgende mathematische Beziehung berechnet:

$$L = 116(Y/Y_n)^{1/3} - 16$$

Die Messung der L-Werte wird mit einem Minolta Spektrophotometer CM-508d durchgeführt. Es werden grundsätzlich zwei Testszenarien für Waschtests zur Ermittlung der Bleichaktivität angewendet. Zum einen Waschtests mit einer kompletten Waschmittelrezeptur ohne TAED (Waschtest mit Vollwaschmittel ohne TAED) und zum anderen ein

vereinfachter Waschtest, der nur Wasserstoffperoxid und Tenside enthält ($H_2O_2$-Test). Folgende Testparameter kommen beim Waschtest mit Vollwaschmittel ohne TAED zur Anwendung:

Volumen der Waschmittellösung: 750 ml
Menge Waschmittel mit TAED: (100 g VWM pro 16 l Flotte, folglich 4,69 g pro 750 ml)
Menge Waschmittel ohne TAED: 4,55 g pro 750 ml
Metallkatalysator: 0,0086 mmol pro Übergangsmetallatom

Temperatur: 30 °C
Waschzeit: 60 min
Spülvolumen: 500 ml
Spülzeit: 15 min
Wasserqualität: künstl. gehärtetes VE-Wasser mit $CaCl_2$ x 2 $H_2O$ (8,73 g pro 25 l) und $MgCl_2$ x 6 $H_2O$ (2,42 g pro 25 l) = 16° dH)
pH-Wert 10,5 (Carbonatpufferlösung)

Die Waschergebnisse für die verschiedenen Übergangsmetallkomplexe in Vollwaschmittel ohne TAED sind in folgender Tabelle gezeigt. Als Vergleichswerte sind der Wert für Vollwaschmittel ohne TAED und ohne Übergangsmetallkomplexe ("ohne Katalysator") sowie der Wert für Vollwaschmittel mit TAED angegeben.

Tabelle 3: Waschtests mit Vollwaschmittel ohne TAED

| | |
|---|---|
| Mn-N-n-Bu-bisq | 76 |
| Mn-N-Me-bisq | 73 |
| ohne Katalysator | 71,7 |
| TAED | 75,8 |

Folgende Testparameter kommen beim vereinfachten Waschtest ($H_2O_2$-Test) zur Anwendung:

Volumen der Waschmittellösung: 750 ml
Menge $H_2O_2$: 10 mmol pro l
Tenside: LAS = 0,58 g; LT07 = 0,12 g
Metallkatalysator: 0,0086 mmol pro Übergangsmetallatom

Temperatur: 30 °C
Waschzeit: 60 min
Wasserqualität: VE-Wasser
pH-Wert 10,5 (Carbonatpufferlösung)

Die Waschergebnisse für die verschiedenen Übergangsmetallkomplexe nach dem vereinfachten Waschtest ($H_2O_2$-Test) sind in folgender Tabelle gezeigt.

Tabelle 4: Vereinfachter Waschtest ($H_2O_2$-Test)

| | |
|---|---|
| Mn-N-n-Bu-bisq | n.d. |
| Mn-N-Me-bisq | 75 |
| Mn-N-H-bisq | 75 |
| Mn-S-bisq | 75 |

**Patentansprüche**

1. Wasch- oder Reinigungsmittel enthaltend Liganden und/oder Metall-Ligand-Komplexe von Liganden der allgemeinen Formel (I)

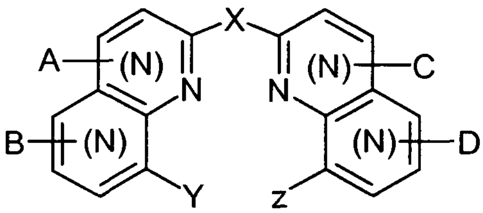

(I),

wobei
X für $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ oder $SiR^3R^4$ steht,
Y und Z unabhängig voneinander für OH, SH, $NH_2$, $NHR^5$ oder $NR^5R^6$ stehen, A, B, C, D, $R^1$, $R^2$, $R^3$ und $R^4$ unabhängig voneinander für Wasserstoff, Alkyl, Trifluormethyl, Cycloalkyl, Cycloalkylalkyl, Alkenyl, Alkinyl, Heteroalkyl, Heterocycloalkyl, Alkoxy, Alkylsulfanyl, Alkylsulfinyl, Alkylsulfonyl, Alkanoyl, Alkanoyloxy, Alkoxycarbonyl, Alkylaminocarbonyl, Alkylsulfanylcarbonyl, Hydroxy, Amino, Aryl, Arylalkyl, Aryloxy, Arylsulfanyl, Arylsulfinyl, Arylsulfonyl, Arylcarbonyl, Arylcarbonyloxy, Aryloxycarbonyl, Arylaminocarbonyl, Arylsulfanylcarbonyl, Heteroaryl, Heteroarylalkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl, Heteroarylsulfanylcarbonyl, Alkoxysulfonyl, Alkoxycarbinol, Ammonium, Hydroxycarbonyl, Alkoxycarbonyl, Aryloxycarbonyl, Amidocarbonyl, Halogen, Nitro, Sulfato, Sulfo, Amidosulfo, Phosphato, Phosphono, Amidophosphono, Formyl, Thioformyl, $-(CH_2-CH_2-O-)_nH$ und $-(CH_2-CH_2-CH_2-O)_nH$ mit n = 1 bis 20,
$R^5$ und $R^6$ unabhängig voneinander für Wasserstoff oder Alkyl stehen,
wobei alle Reste des sich so ergebenden Moleküls jeweils unabhängig voneinander gegebenenfalls auch ein- oder mehrfach substituiert sein können, wobei (N) bedeutet, dass optional ein oder zwei CH-Gruppen des entsprechenden Arylrests durch N ersetzt sein können.

2. Wasch- oder Reinigungsmittel nach Anspruch 1, **dadurch gekennzeichnet, dass** es sich bei den Liganden um Liganden der allgemeinen Formel (II)

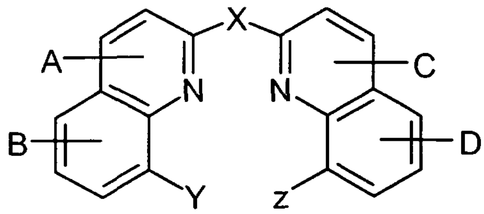

(II)

handelt.

3. Wasch- oder Reinigungsmittel nach Anspruch 2, **dadurch gekennzeichnet, dass** es sich um Liganden der allgemeinen Formel (III)

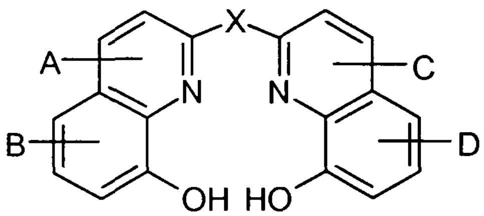

(III)

handelt.

4. Wasch- oder Reinigungsmittel nach einem der Ansprüche 1 bis 3, **dadurch gekennzeichnet, dass** $R^1$ und $R^2$ unabhängig voneinander für Wasserstoff, Alkyl, Cycloalkyl, Cycloalkylalkyl, Alkenyl, Alkinyl, Heteroalkyl, Heterocycloalkyl, Alkanoyl, Alkoxycarbonyl, Alkylaminocarbonyl, Alkylsulfanylcarbonyl, Aryl, Arylalkyl, Arylcarbonyl, Aryloxycarbonyl, Arylaminocarbonyl, Arylsulfanylcarbonyl, Heteroaryl, Heteroarylalkyl, Heteroarylcarbonyl, Heteroaryloxycarbonyl, Heteroarylaminocarbonyl, Heteroarylsulfanylcarbonyl, Trifluormethyl, Formyl, $-(CH_2-CH_2-O-)_nH$ oder $-(CH_2-CH_2-CH_2-O)_nH$ mit n = 1 bis 20, stehen, wobei alle Reste des sich so ergebenden Moleküls jeweils unabhängig voneinander gegebenenfalls auch ein- oder mehrfach substituiert sein können.

**5.** Wasch- oder Reinigungsmittel nach einem der Ansprüche 1 bis 4, **dadurch gekennzeichnet, dass** X für $NR^1$, $NR^1R^{2(+)}$, $PR^1$ oder $PR^1R^{2(+)}$ steht.

**6.** Wasch- oder Reinigungsmittel nach einem der Ansprüche 1, 2, 4 5, und **dadurch gekennzeichnet, dass** Y und Z für OH stehen.

**7.** Wasch- oder Reinigungsmittel nach einem der Ansprüche 1 bis 6, **dadurch gekennzeichnet, dass**
X für $NR^1$, $NR^1R^{2(+)}$, $PR^1$ oder $PR^1R^{2(+)}$ steht,
Y und Z für OH stehen,
$R^1$ für Wasserstoff, $C_{1-6}$-Alkyl, $-(CH_2-CH_2-O-)_nH$ oder $-(CH_2-CH_2-CH_2-O)_nH$ mit n = 1 bis 20 steht,
A, B, C und D unabhängig voneinander für Wasserstoff, Alkyl, Ammonium, Hydroxycarbonyl, Alkoxycarbonyl, Aryloxycarbonyl, Amidocarbonyl, Halogen, Nitro, Sulfato, Sulfo, Amidosulfo, Phosphato, Phosphono, Amidophosphono, Hydroxy, Alkoxy oder Amino stehen,
wobei alle Reste des sich so ergebenden Moleküls jeweils unabhängig voneinander gegebenenfalls auch ein- oder mehrfach substituiert sein können.

**8.** Wasch- oder Reinigungsmittel nach einem der Ansprüche 1 bis 7, **dadurch gekennzeichnet, dass** mindestens einer der Reste A, B, C, D und X eine Gruppe ausgewählt aus Ammonium, Hydroxycarbonyl, Alkoxycarbonyl, Aryloxycarbonyl, Amidocarbonyl, Halogen, Nitro, Sulfato, Sulfo, Amidosulfo, Phosphato, Phosphono, Amidophosphono, Hydroxy, Alkoxy, Amino und Polyoxyethylen umfasst.

**9.** Wasch- oder Reinigungsmittel nach einem der Ansprüche 1 bis 8, **dadurch gekennzeichnet, dass** das Metall des Metall-Ligand-Komplexes ausgewählt ist aus Ag, Al, Ce, Co, Cu, Fe, Mo, Mn, Ni, Pb, Re, Ti, V und Zn in beliebigen Oxidationsstufen.

**10.** Verwendung von Wasch- oder Reinigungsmitteln nach einem der Ansprüche 1 bis 9 zur Reinigung textiler Flächengebilde oder harter Oberflächen.

**11.** Verwendung von Liganden und/oder Ligand-Metall-Komplexen von Liganden der allgemeinen Formel (I)

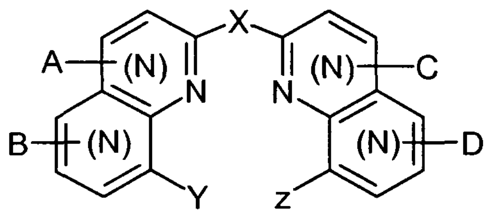
(I),

wobei
X für $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ oder $SiR^3R^4$ steht,
Y und Z unabhängig voneinander für OH, SH, $NH_2$, $NHR^5$ oder $NR^5R^6$ stehen,
A, B, C, D, $R^1$, $R^2$, $R^3$ und $R^4$ unabhängig voneinander für Wasserstoff, Alkyl, Trifluormethyl, Cycloalkyl, Cycloalkylalkyl, Alkenyl, Alkinyl, Heteroalkyl, Heterocycloalkyl, Alkoxy, Alkylsulfanyl, Alkylsulfinyl, Alkylsulfonyl, Alkanoyl, Alkanoyloxy, Alkoxycarbonyl, Alkylaminocarbonyl, Alkylsulfanylcarbonyl, Hydroxy, Amino, Aryl, Arylalkyl, Aryloxy, Arylsulfanyl, Arylsulfinyl, Arylsulfonyl, Arylcarbonyl, Arylcarbonyloxy, Aryloxycarbonyl, Arylaminocarbonyl, Arylsulfanylcarbonyl, Heteroaryl, Heteroarylalkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl, Heteroarylsulfanylcarbonyl, Alkoxysulfonyl, Alkoxycarbinol, Ammonium, Hydroxycarbonyl, Alkoxycarbonyl, Aryloxycarbonyl, Amidocarbonyl, Halogen, Nitro, Sulfato, Sulfo, Amidosulfo, Phosphato, Phosphono, Amidophosphono, Formyl, Thioformyl, $-(CH_2-CH_2-O-)_nH$ und $-(CH_2-CH_2-CH_2-O)_nH$ mit n = 1 bis 20,
$R^5$ und $R^6$ unabhängig voneinander für Wasserstoff oder Alkyl stehen,
wobei alle Reste des sich so ergebenden Moleküls jeweils unabhängig voneinander gegebenenfalls auch ein- oder mehrfach substituiert sein können.

zur Reinigung textiler Flächengebilde oder harter Oberflächen.

**12.** Verwendung von Liganden und/oder Ligand-Metall-Komplexen von Liganden der allgemeinen Formel (I)

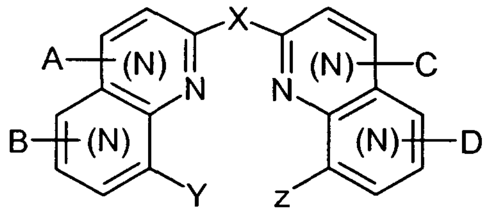

(I),

wobei
X für $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ oder $SiR^3R^4$ steht,
Y und Z unabhängig voneinander für OH, SH, $NH_2$, $NHR^5$ oder $NR^5R^6$ stehen,
A, B, C, D, $R^1$, $R^2$, $R^3$ und $R^4$ unabhängig voneinander für Wasserstoff, Alkyl, Trifluormethyl, Cycloalkyl, Cycloalkylalkyl, Alkenyl, Alkinyl, Heteroalkyl, Heterocycloalkyl, Alkoxy, Alkylsulfanyl, Alkylsulfinyl, Alkylsulfonyl, Alkanoyl, Alkanoyloxy, Alkoxycarbonyl, Alkylaminocarbonyl, Alkylsulfanylcarbonyl, Hydroxy, Amino, Aryl, Arylalkyl, Aryloxy, Arylsulfanyl, Arylsulfinyl, Arylsulfonyl, Arylcarbonyl, Arylcarbonyloxy, Aryloxycarbonyl, Arylaminocarbonyl, Arylsulfanylcarbonyl, Heteroaryl, Heteroarylalkyl, Heteroaryloxy, Heteroarylamino, Heteroarylsulfanyl, Heteroarylsulfonyl, Heteroarylsulfoxidyl, Heteroarylcarbonyl, Heteroarylcarbonyloxy, Heteroaryloxycarbonyl, Heteroarylaminocarbonyl, Heteroarylsulfanylcarbonyl, Alkoxysulfonyl, Alkoxycarbinol, Ammonium, Hydroxycarbonyl, Alkoxycarbonyl, Aryloxycarbonyl, Amidocarbonyl, Halogen, Nitro, Sulfato, Sulfo, Amidosulfo, Phosphato, Phosphono, Amidophosphono, Formyl, Thioformyl, $-(CH_2-CH_2-O-)_nH$ und $-(CH_2-CH_2-CH_2-O)_nH$ mit n = 1 bis 20,
$R^5$ und $R^6$ unabhängig voneinander für Wasserstoff oder Alkyl stehen,
wobei alle Reste des sich so ergebenden Moleküls jeweils unabhängig voneinander gegebenenfalls auch ein- oder mehrfach substituiert sein können.
zum Bleichen von Zellstoff und/oder Roh-Baumwolle.

## Claims

**1.** A washing or cleaning agent containing ligands and/or metal-ligand complexes of ligands of the general formula (I)

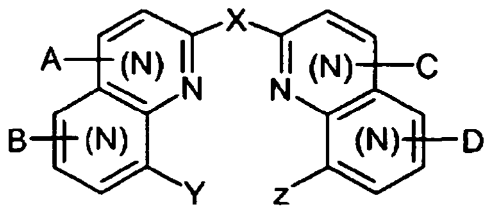

(I),

wherein
X denotes $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ or $SiR^3R^4$,
Y and Z mutually independently denote OH, SH, $NH_2$, $NHR^5$ or $NR^5R^6$, A, B, C, D, $R^1$, $R^2$, $R^3$ and $R^4$ mutually independently denote hydrogen, alkyl, trifluoromethyl, cycloalkyl, cycloalkylalkyl, alkenyl, alkynyl, heteroalkyl, heterocycloalkyl, alkoxy, alkylsulfanyl, alkylsulfinyl, alkylsulfonyl, alkanoyl, alkanoyloxy, alkoxycarbonyl, alkylaminocarbonyl, alkylsulfanylcarbonyl, hydroxy, amino, aryl, arylalkyl, aryloxy, arylsulfanyl, arylsulfinyl, arylsulfonyl, arylcarbonyl, arylcarbonyloxy, aryloxycarbonyl, arylaminocarbonyl, arylsulfanylcarbonyl, heteroaryl, heteroarylalkyl, heteroaryloxy, heteroarylamino, heteroarylsulfanyl, heteroarylsulfonyl, heteroarylsulfoxidyl, heteroarylcarbonyl, heteroarylcarbonyloxy, heteroaryloxycarbonyl, heteroarylaminocarbonyl, heteroarylsulfanylcarbonyl, alkoxysulfonyl, alkoxycarbinol, ammonium, hydroxycarbonyl, alkoxycarbonyl, aryloxycarbonyl, amidocarbonyl, halogen, nitro, sulfato, sulfo, amidosulfo, phosphato, phosphono, amidophosphono, formyl, thioformyl, $-(CH_2-CH_2-O-)_nH$ and $-(CH_2-CH_2-CH_2-O)_nH$ with n = 1 to 20,
$R^5$ and $R^6$ mutually independently denote hydrogen or alkyl, wherein all the residues of the resultant molecule may in each case mutually independently optionally also be mono- or polysubstituted, wherein (N) means that ideally

one or two CH groups of the corresponding aryl residue may be replaced by N.

**2.** A washing or cleaning agent according to claim 1, **characterised in that** the ligands comprise ligands of the general formula (II)

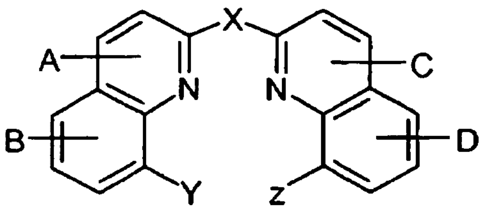

(II) .

**3.** A washing or cleaning agent according to claim 2, **characterised in that** the ligands are of the general formula (III)

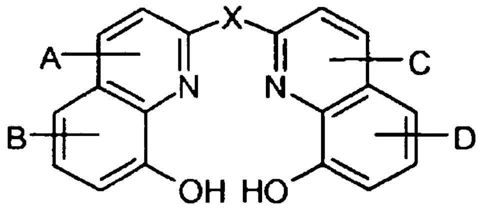

(III) .

**4.** A washing or cleaning agent according to any one of claims 1 to 3, **characterised in that** $R^1$ and $R^2$ mutually independently denote hydrogen, alkyl, cycloalkyl, cycloalkylalkyl, alkenyl, alkynyl, heteroalkyl, heterocycloalkyl, alkanoyl, alkoxycarbonyl, alkylaminocarbonyl, alkylsulfanylcarbonyl, aryl, arylalkyl, arylcarbonyl, aryloxycarbonyl, arylaminocarbonyl, arylsulfanylcarbonyl, heteroaryl, heteroarylalkyl, heteroarylcarbonyl, heteroaryloxycarbonyl, heteroarylaminocarbonyl, heteroarylsulfanylcarbonyl, trifluoromethyl, formyl, $-(CH_2-CH_2-O-)_nH$ or $-(CH_2-CH_2-CH_2-O)_nH$ with n = 1 to 20, wherein all the residues of the resultant molecule may in each case mutually independently optionally also be mono- or polysubstituted.

**5.** A washing or cleaning agent according to any one of claims 1 to 4, **characterised in that** X denotes $NR^1$, $NR^1R^{2(+)}$, $PR^1$ or $PR^1R^{2(+)}$.

**6.** A washing or cleaning agent according to any one of claims 1, 2, 4 and 5, **characterised in that** Y and Z denote OH.

**7.** A washing or cleaning agent according to any one of claims 1 to 6,
**characterised in that**
X denotes $NR^1$, $NR^1R^{2(+)}$ $PR^1$ or $PR^1R^{2(+)}$,
Y and Z denote OH,
$R^1$ denotes hydrogen, $C_{1-6}$ alkyl, $-(CH_2-CH_2-O-)_nH$ or $-(CH_2-CH_2-CH_2-O)_nH$ with n = 1 to 20,
A, B, C and D mutually independently denote hydrogen, alkyl, ammonium, hydroxycarbonyl, alkoxycarbonyl, aryloxycarbonyl, amidocarbonyl, halogen, nitro, sulfato, sulfo, amidosulfo, phosphato, phosphono, amidophosphono, hydroxy, alkoxy or amino,
wherein all the residues of the resultant molecule may in each case mutually independently optionally also be mono- or polysubstituted.

**8.** A washing or cleaning agent according to any one of claims 1 to 7, **characterised in that** at least one the residues A, B, C, D and X comprises a group selected from ammonium, hydroxycarbonyl, alkoxycarbonyl, aryloxycarbonyl, amidocarbonyl, halogen, nitro, sulfato, sulfo, amidosulfo, phosphato, phosphono, amidophosphono, hydroxy, alkoxy, amino and polyoxyethylene.

**9.** A washing or cleaning agent according to any one of claims 1 to 8, **characterised in that** the metal of the metal-ligand complex is selected from Ag, Al, Ce, Co, Cu, Fe, Mo, Mn, Ni, Pb, Re, Ti, V and Zn in any oxidation state.

**10.** Use of washing or cleaning agents according to any one of claims 1 to 9 for cleaning textile fabrics or hard surfaces.

**11.** Use of ligands and/or ligand-metal complexes of ligands of the general formula (I)

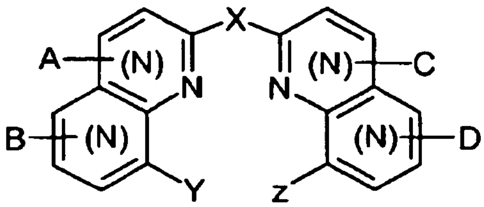

(I),

wherein
X denotes $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ or $SiR^3R^4$,
Y and Z mutually independently for denote OH, SH, $NH_2$, $NHR^5$ or $NR^5R^6$,
A, B, C, D, $R^1$, $R^2$, $R^3$ and $R^4$ mutually independently denote hydrogen, alkyl, trifluoromethyl, cycloalkyl, cycloalkylalkyl, alkenyl, alkynyl, heteroalkyl, heterocycloalkyl, alkoxy, alkylsulfanyl, alkylsulfinyl, alkylsulfonyl, alkanoyl, alkanoyloxy, alkoxycarbonyl, alkylaminocarbonyl, alkylsulfanylcarbonyl, hydroxy, amino, aryl, arylalkyl, aryloxy, arylsulfanyl, arylsulfinyl, arylsulfonyl, arylcarbonyl, arylcarbonyloxy, aryloxycarbonyl, arylaminocarbonyl, arylsulfanylcarbonyl, heteroaryl, heteroarylalkyl, heteroaryloxy, heteroarylamino, heteroarylsulfanyl, heteroarylsulfonyl, heteroarylsulfoxidyl, heteroarylcarbonyl, heteroarylcarbonyloxy, heteroaryloxycarbonyl, heteroarylaminocarbonyl, heteroarylsulfanylcarbonyl, alkoxysulfonyl, alkoxycarbinol, ammonium, hydroxycarbonyl, alkoxycarbonyl, aryloxycarbonyl, amidocarbonyl, halogen, nitro, sulfato, sulfo, amidosulfo, phosphato, phosphono, amidophosphono, formyl, thioformyl, $-(CH_2-CH_2-O-)_nH$ and $-(CH_2-CH_2-CH_2-O)_nH$ with n = 1 to 20,
$R^5$ and $R^6$ mutually independently denote hydrogen or alkyl, wherein all the residues of the resultant molecule may in each case mutually independently optionally also be mono- or polysubstituted,
for cleaning textile fabrics or hard surfaces.

**12.** Use of ligands and/or ligand-metal complexes of ligands of the general formula (I)

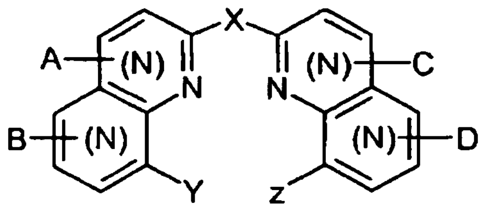

(I),

wherein
X denotes $NR^1$, $NR^1R^{2(+)}$, $PR^1$, $PR^1R^{2(+)}$, $P(O)R^1$, O, S, $BR^1$, $BR^1R^{2(-)}$, $CR^3R^4$ or $SiR^3R^4$,
Y and Z mutually independently denote OH, SH, $NH_2$, $NHR^5$ or $NR^5R^6$,
A, B, C, D, $R^1$, $R^2$, $R^3$ and $R^4$ mutually independently denote hydrogen, alkyl, trifluoromethyl, cycloalkyl, cycloalkylalkyl, alkenyl, alkynyl, heteroalkyl, heterocycloalkyl, alkoxy, alkylsulfanyl, alkylsulfinyl, alkylsulfonyl, alkanoyl, alkanoyloxy, alkoxycarbonyl, alkylaminocarbonyl, alkylsulfanylcarbonyl, hydroxy, amino, aryl, arylalkyl, aryloxy, arylsulfanyl, arylsulfinyl, arylsulfonyl, arylcarbonyl, arylcarbonyloxy, aryloxycarbonyl, arylaminocarbonyl, arylsulfanylcarbonyl, heteroaryl, heteroarylalkyl, heteroaryloxy, heteroarylamino, heteroarylsulfanyl, heteroarylsulfonyl, heteroarylsulfoxidyl, heteroarylcarbonyl, heteroarylcarbonyloxy, heteroaryloxycarbonyl, heteroarylaminocarbonyl, heteroarylsulfanylcarbonyl, alkoxysulfonyl, alkoxycarbinol, ammonium, hydroxycarbonyl, alkoxycarbonyl, aryloxycarbonyl, amidocarbonyl, halogen, nitro, sulfato, sulfo, amidosulfo, phosphato, phosphono, amidophosphono, formyl, thioformyl, $-(CH_2-CH_2-O-)_nH$ and $-(CH_2-CH_2-CH_2-O)_nH$ with n = 1 to 20,
$R^5$ and $R^6$ mutually independently denote hydrogen or alkyl,
wherein all the residues of the resultant molecule may in each case mutually independently optionally also be mono- or polysubstituted,
for bleaching woodpulp and/or raw cotton.

## Revendications

1. Agent de lavage ou de nettoyage contenant des ligands et/ou des complexes métaux-ligands de ligands répondant à la formule générale (I)

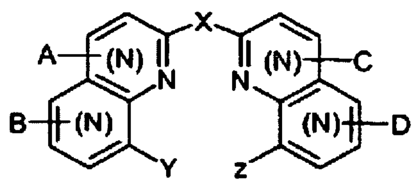

(I).

dans laquelle
X représente un groupe $NR^1$, un groupe $NR^1R^{2(+)}$, un groupe $PR^1$, un groupe $PR^1R^{2(+)}$, un groupe $P(O)R^1$, un atome d'oxygène, un atome de soufre, un groupe $BR^1$, un groupe $BR^1R^{2(-)}$, un groupe $CR^3R^4$ ou un groupe $SiR^3R^4$;
Y et Z représentent, indépendamment l'un de l'autre, un groupe OH, un groupe SH, un groupe $NH_2$, un groupe $NHR^5$ ou un groupe $NR^5R^6$;
A, B, C, D, $R^1$, $R^2$, $R^3$ et $R^4$ représentent, indépendamment l'un de l'autre, un atome d'hydrogène, un groupe alkyle, un groupe trifluorométhyle, un groupe cycloalkyle, un groupe cycloalkylalkyle, un groupe alcényle, un groupe alcynyle, un groupe hétéroalkyle, un groupe hétérocycloalkyle, un groupe alcoxy, un groupe alkylsulfanyle, un groupe alkylsulfynyle, un groupe alkylsulfonyle, un groupe alcanoyle, un groupe alcanoyloxy, un groupe alcoxycarbonyle, un groupe alkylaminocarbonyle, un groupe alkylsulfanylcarbonyle, un groupe hydroxyle, un groupe amino, un groupe aryle, un groupe arylalkyle, un groupe aryloxy, un groupe arylsulfanyle, un groupe arylsulfynyle, un groupe arylsulfonyle, un groupe arylcarbonyle, un groupe arylcarbonyloxy, un groupe aryloxycarbonyle, un groupe arylaminocarbonyle, un groupe arylsulfanylcarbonyle, un groupe hétéroaryle, un groupe hétéroarylalkyle, un groupe hétéroaryloxy, un groupe hétéroarylamino, un groupe hétéroarylsulfanyle, un groupe hétéroarylsulfonyle, un groupe hétéroarylsulfoxydyle, un groupe hétéroarylcarbonyle, un groupe hétéroarylcarbonyloxy, un groupe hétéroaryloxycarbonyle, un groupe hétéroarylaminocarbonyle, un groupe hétéroarylsulfanylcarbonyle, un groupe alcoxysulfonyle, un groupe alcoxycarbinol, un groupe ammonium, un groupe hydroxycarbonyle, un groupe alcoxycarbonyle, un groupe aryloxycarbonyle, un groupe amidocarbonyle, un atome d'halogène, un groupe nitro, un groupe sulfato, un groupe sulfo, un groupe amidosulfo, un groupe phosphato, un groupe phosphono, un groupe amidophosphono, un groupe formyle, un groupe thioformyle, un groupe $-(CH_2-CH_2-O-)_nH$ et un groupe $-(CH_2-CH_2-CH_2-O)_nH$ avec n = de 1 à 20 ;
$R^5$ et $R^6$ représentent, indépendamment l'un de l'autre, un atome d'hydrogène ou un groupe alkyle ;
tous les résidus de la molécule que l'on obtient de la sorte pouvant, à titre facultatif, chaque fois de manière respectivement indépendante, également être substitués une ou plusieurs fois, (N) indiquant que, dans des conditions optimales, un ou deux groupes CH du résidu aryle correspondant peuvent être remplacés par un atome d'azote.

2. Agent de lavage ou de nettoyage selon la revendication 1, **caractérisé en ce qu'**il s'agit, en ce qui concerne les ligands, de ligands répondant à la formule générale (II)

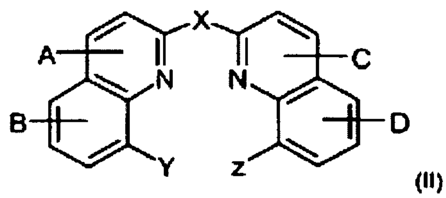

(II)

3. Agent de lavage ou de nettoyage selon la revendication 2, **caractérisé en ce qu'**il s'agit, en ce qui concerne les ligands, de ligands répondant à la formule générale (III)

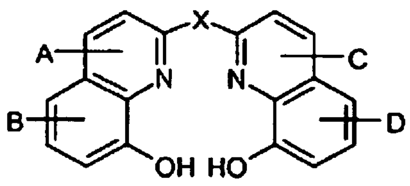

(III)

4. Agent de lavage ou de nettoyage selon l'une quelconque des revendications 1 à 3, **caractérisé en ce que** $R^1$ et $R^2$ représentent, indépendamment l'un de l'autre, un atome d'hydrogène, un groupe alkyle, un groupe cycloalkyle, un groupe cycloalkylalkyle, un groupe alcényle, un groupe alcynyle, un groupe hétéroalkyle, un groupe hétérocycloalkyle, un groupe alcanoyle, un groupe alcoxycarbonyle, un groupe alkylaminocarbonyle, un groupe alkylsulfanylcarbonyle, un groupe aryle, un groupe arylalkyle, un groupe arylcarbonyle, un groupe aryloxycarbonyle, un groupe arylaminocarbonyle, un groupe arylsulfanylcarbonyle, un groupe hétéroaryle, un groupe hétéroarylalkyle, un groupe hétéroarylcarbonyle, un groupe hétéroaryloxycarbonyle, un groupe hétéroarylaminocarbonyle; un groupe hétéroarylsulfanylcarbonyle, un groupe trifluorométhyle, un groupe formyle, un groupe - $(CH_2\text{-}CH_2\text{-}O\text{-})_nH$ ou un groupe $-(CH_2\text{-}CH_2\text{-}CH_2\text{-}O)_nH$ avec n = de 1 à 20 ; tous les résidus de la molécule que l'on obtient de la sorte pouvant, à titre facultatif, chaque fois de manière respectivement indépendante, également être substitués une ou plusieurs fois.

5. Agent de lavage ou de nettoyage selon l'une quelconque des revendications 1 à 4, **caractérisé en ce que** X représente un groupe $NR^1$, un groupe $NR^1R^{2(+)}$, un groupe $PR^1$ ou un groupe $PR^1R^{2(+)}$.

6. Agent de lavage ou de nettoyage selon l'une quelconque des revendications 1, 2, 4 et 5, **caractérisé en ce que** Y et Z représentent un groupe OH.

7. Agent de lavage ou de nettoyage selon l'une quelconque des revendications 1 à 6, **caractérisé en ce que**
X représente un groupe $NR^1$, un groupe $NR^1R^{2(+)}$, un groupe $PR^1$ ou un groupe $PR^1R^{2(+)}$;
Y et Z représentent un groupe OH ;
$R^1$ représente un atome d'hydrogène, un groupe alkyle en $C_1$-$C_6$, un groupe $-(CH_2\text{-}CH_2\text{-}O\text{-})_nH$ et un groupe $-(CH_2\text{-}CH_2\text{-}CH_2\text{-}O)_nH$ avec n = de 1 à 20 ;
A, B, C et D représentent, indépendamment l'un de l'autre, un atome d'hydrogène, un groupe alkyle, un groupe ammonium, un groupe hydroxycarbonyle, un groupe alcoxycarbonyle, un groupe aryloxycarbonyle, un groupe amidocarbonyle, un atome d'halogène, un groupe nitro, un groupe sulfato, un groupe sulfo, un groupe amidosulfo, un groupe phosphato, un groupe phosphono, un groupe amidophosphono, un groupe hydroxyle, un groupe alcoxy ou un groupe amino,
tous les résidus de la molécule que l'on obtient de la sorte pouvant, à titre facultatif, chaque fois de manière respectivement indépendante, également être substitués une ou plusieurs fois.

8. Agent de lavage ou de nettoyage selon l'une quelconque des revendications 1 à 7, **caractérisé en ce qu'**au moins un des résidus A, B, C, D et X comprend un groupe choisi parmi un groupe ammonium, un groupe hydroxycarbonyle, un groupe alcoxycarbonyle, un groupe aryloxycarbonyle, un groupe amidocarbonyle, un atome d'halogène, un groupe nitro, un groupe sulfato, un groupe sulfo, un groupe amidosulfo, un groupe phosphato, un groupe phosphono, un groupe amidophosphono, un groupe hydroxyle, un groupe alcoxy, un groupe amino et un groupe polyoxyéthylène.

9. Agent de lavage ou de nettoyage selon l'une quelconque des revendications 1 à 8, **caractérisé en ce que** le métal du complexe métal-ligand est choisi parmi Ag, Al, Ce, Co, Cu, Fe, Mo, Mn, Ni, Pb, Re, Ti, V et Zn dans n'importe quel état d'oxydation.

10. Utilisation d'agents de lavage ou de nettoyage selon l'une quelconque des revendications 1 à 9, pour le nettoyage de produits plats textiles ou de surfaces dures.

11. Utilisation de ligands et/ou de complexes ligands-métaux de ligands répondant à la formule générale (I)

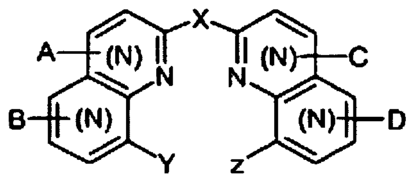

(I).

dans laquelle
X représente un groupe $NR^1$, un groupe $NR^1R^{2(+)}$, un groupe $PR^1$, un groupe $PR^1R^{2(+)}$, un groupe $P(O)R^1$, un atome d'oxygène, un atome de soufre, un groupe $BR^1$, un groupe $BR^1R^{2(-)}$, un groupe $CR^3R^4$ ou un groupe $SiR^3R^4$ ;
Y et Z représentent, indépendamment l'un de l'autre, un groupe OH, un groupe SH, un groupe $NH_2$, un groupe $NHR^5$ ou un groupe $NR^5R^6$ ;
A, B, C, D, $R^1$, $R^2$, $R^3$ et $R^4$ représentent, indépendamment l'un de l'autre, un atome d'hydrogène, un groupe alkyle, un groupe trifluorométhyle, un groupe cycloalkyle, un groupe cycloalkylalkyle, un groupe alcényle, un groupe alcynyle, un groupe hétéroalkyle, un groupe hétérocycloalkyle, un groupe alcoxy, un groupe alkylsulfanyle, un groupe alkylsulfynyle, un groupe alkylsulfonyle, un groupe alcanoyle, un groupe alcanoyloxy, un groupe alcoxycarbonyle, un groupe alkylaminocarbonyle, un groupe alkylsulfanylcarbonyle, un groupe hydroxyle, un groupe amino, un groupe aryle, un groupe arylalkyle, un groupe aryloxy, un groupe arylsulfanyle, un groupe arylsulfynyle, un groupe arylsulfonyle, un groupe arylcarbonyle, un groupe arylcarbonyloxy, un groupe aryloxycarbonyle, un groupe arylaminocarbonyle, un groupe arylsulfanylcarbonyle, un groupe hétéroaryle, un groupe hétéroarylalkyle, un groupe hétéroaryloxy, un groupe hétéroarylamino, un groupe hétéroarylsulfanyle, un groupe hétéroarylsulfonyle, un groupe hétéroarylsulfoxydyle, un groupe hétéroarylcarbonyle, un groupe hétéroarylcarbonyloxy, un groupe hétéroaryloxycarbonyle, un groupe hétéroarylaminocarbonyle, un groupe hétéroarylsulfanylcarbonyle, un groupe alcoxysulfonyle, un groupe alcoxycarbinol, un groupe ammonium, un groupe hydroxycarbonyle, un groupe alcoxycarbonyle, un groupe aryloxycarbonyle, un groupe amidocarbonyle, un atome d'halogène, un groupe nitro, un groupe sulfato, un groupe sulfo, un groupe amidosulfo, un groupe phosphato, un groupe phosphono, un groupe amidophosphono, un groupe formyle, un groupe thioformyle, un groupe $-(CH_2-CH_2-O-)_nH$ et un groupe $-(CH_2-CH_2-CH_2-O)_nH$ avec n = de 1 à 20 ;
$R^5$ et $R^6$ représentent, indépendamment l'un de l'autre, un atome d'hydrogène ou un groupe alkyle ;
tous les résidus de la molécule que l'on obtient de la sorte pouvant, à titre facultatif, chaque fois de manière respectivement indépendante, également être substitués une ou plusieurs fois,
pour le nettoyage de produits plats textiles ou de surfaces dures.

12. Utilisation de ligands et/ou de complexes ligands-métaux de ligands répondant à la formule générale (I)

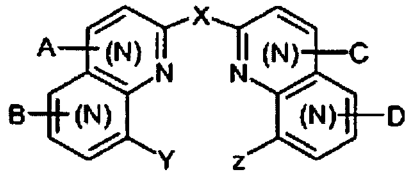

(I).

dans laquelle
X représente un groupe $NR^1$, un groupe $NR^1R^{2(+)}$, un groupe $PR^1$, un groupe $PR^1R^{2(+)}$, un groupe $P(O)R^1$, un atome d'oxygène, un atome de soufre, un groupe $BR^1$, un groupe $BR^1R^{2(-)}$, un groupe $CR^3R^4$ ou un groupe $SiR^3R^4$ ;
Y et Z représentent, indépendamment l'un de l'autre, un groupe OH, un groupe SH, un groupe $NH_2$, un groupe $NHR^5$ ou un groupe $NR^5R^6$ ;
A, B, C, D, $R^1$, $R^2$, $R^3$ et $R^4$ représentent, indépendamment l'un de l'autre, un atome d'hydrogène, un groupe alkyle, un groupe trifluorométhyle, un groupe cycloalkyle, un groupe cycloalkylalkyle, un groupe alcényle, un groupe alcynyle, un groupe hétéroalkyle, un groupe hétérocycloalkyle, un groupe alcoxy, un groupe alkylsulfanyle, un groupe alkylsulfynyle, un groupe alkylsulfonyle, un groupe alcanoyle, un groupe alcanoyloxy, un groupe alcoxycarbonyle, un groupe alkylaminocarbonyle, un groupe alkylsulfanylcarbonyle, un groupe hydroxyle, un groupe amino, un groupe aryle, un groupe arylalkyle, un groupe aryloxy, un groupe arylsulfanyle, un groupe arylsulfynyle, un groupe arylsulfonyle, un groupe arylcarbonyle, un groupe arylcarbonyloxy, un groupe aryloxycarbonyle, un groupe arylaminocarbonyle, un groupe arylsulfanylcarbonyle, un groupe hétéroaryle, un groupe hétéroarylalkyle, un groupe hétéroaryloxy, un groupe hétéroarylamino, un groupe hétéroarylsulfanyle, un groupe hétéroarylsulfonyle, un groupe hétéroarylsulfoxydyle, un groupe hétéroarylcarbonyle, un groupe hétéroarylcarbonyloxy, un groupe hétéroaryloxycarbonyle, un groupe hétéroarylaminocarbonyle, un groupe hétéroarylsulfanylcarbonyle, un groupe alcoxysulfonyle, un groupe alcoxycarbinol, un groupe ammonium, un groupe hydroxycarbonyle, un groupe alcoxycarbonyle, un groupe aryloxycarbonyle, un groupe amidocarbonyle, un atome d'halogène, un groupe nitro, un groupe sulfato, un groupe sulfo, un groupe amidosulfo, un groupe phosphato, un groupe phosphono, un groupe amidophosphono, un groupe formyle, un groupe thioformyle, un groupe $-(CH_2-CH_2-O-)_nH$ et un groupe $-(CH_2-CH_2-CH_2-O)_nH$ avec n = de 1 à 20 ;

$R^5$ et $R^6$ représentent, indépendamment l'un de l'autre, un atome d'hydrogène ou un groupe alkyle ;

tous les résidus de la molécule que l'on obtient de la sorte pouvant, à titre facultatif, chaque fois de manière respectivement indépendante, également être substitués une ou plusieurs fois,

pour le blanchiment de cellulose et/ou de coton brut.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19825737
- DE 2650764
- WO 0458955 A
- DE 102005053529
- WO 2008135337 A
- WO 9604940 A


### In der Beschreibung aufgeführte Nicht-Patentliteratur


- **AGUSTIN et al.** *J. Organomet. Chem.,* 1999, vol. 592, 1-10
- **FAGAN et al.** *J. Am. Chem. Soc.,* 2000, vol. 122, 5043-5051
- **STOCKWELL et al.** *J. Am. Chem. Soc.,* 1999, vol. 121, 10662-10663
- **WANG et al.** *Inorganica Chimica Acta,* 2001, vol. 321, 215-220